(19)

Europäisches
Patentamt
European
Patent Office
Office européen
des brevets

(11) **EP 4 706 672 A1**

(12) **EUROPEAN PATENT APPLICATION**
published in accordance with Art. 153(4) EPC

(43) Date of publication:
**11.03.2026  Bulletin 2026/11**

(21) Application number: **24796163.4**

(22) Date of filing: **25.04.2024**

(51) International Patent Classification (IPC):
***A61K 39/00*** (2006.01)          ***A61K 35/74*** (2015.01)
***A61K 31/07*** (2006.01)          ***A61K 47/34*** (2017.01)
***A61P 35/00*** (2006.01)

(52) Cooperative Patent Classification (CPC):
Y02A 50/30

(86) International application number:
**PCT/CN2024/089764**

(87) International publication number:
**WO 2024/222787 (31.10.2024 Gazette 2024/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **27.04.2023  CN 202310469235**

(71) Applicant: **Pugong Biotech (Hangzhou) Co., Ltd.
Hangzhou, Zhejiang 311103 (CN)**

(72) Inventors:
• **CHANG, Jian
  Hangzhou, Zhejiang 311103 (CN)**
• **CHEN, Lei
  Hangzhou, Zhejiang 311103 (CN)**
• **GAO, Wenbin
  Hangzhou, Zhejiang 311103 (CN)**

(74) Representative: **Canzler & Bergmeier
Patentanwälte
Partnerschaft mbB
Despag-Straße 6
85055 Ingolstadt (DE)**

(54) **PHARMACEUTICAL COMPOSITION, AND PREPARATION METHOD THEREFOR AND USE THEREOF**

(57)    A pharmaceutical composition, and a preparation method therefor and a use thereof. The pharmaceutical composition comprises a first active ingredient, a second active ingredient, and a pharmaceutically acceptable carrier or adjuvant. The first active ingredient is a microbial agent comprising any one or more of staphylococcus aureus, bordetella pertussis, diphtheria toxoid, tetanus toxoid, typhoid bacillus or salmonella paratyphi. The second active ingredient comprises polyinosinic acid, polycytidylic acid, and a vitamin. The pharmaceutical composition relates to an artificial active immunization therapy for tumors, and can "stimulate" the entire immune system, so that the therapy of using bacteria to stimulate a human immune system so as to kill cancer cells is quite stable and reliable, and the lives of cancer patients can be significantly saved and prolonged. Additionally, the pharmaceutical composition has extremely high safety, extremely small toxic and side effects, and low preparation costs.

EP 4 706 672 A1

**Description**

**FIELD OF TECHNOLOGY**

The present disclosure relates to the technical field of medicine, and in particular to a pharmaceutical composition, preparation method and use thereof.

**BACKGROUND**

Cancer is a leading threat to human health and life. The conventional treatment methods for cancer include surgery, radiotherapy, chemotherapy, and targeted therapy. Over the past decade, tumor immunotherapy, mainly passive immunotherapy, has gradually become an alternative approach for cancer treatment.

However, none of these therapies can fundamentally and systematically address the issue. Surgery and radiotherapy can only eliminate localized lesions. Except in cases of early-stage primary tumors, they are generally incapable of eradicating disseminated disease. Chemotherapy, as a systemic treatment, is currently the primary option for advanced-stage tumors. While chemotherapy effectively kills cancer cells, it also non-selectively damages normal cells, causing irreversible harm to the patient. Additionally, chemotherapy can impair the immune system in some patients, preventing recovery or reconstruction, resulting in the near-complete loss of immune surveillance mechanisms. Consequently, a significant number of patients die from tumor metastasis or disease progression. The specificity of targeted therapy limits its scope of action, and drug resistance remains a major obstacle. Currently, mainstream passive immunotherapy is expensive, has a narrow range of indications, and does not provide a significant improvement in efficacy compared to other treatment methods. It also exhibits notable toxic side effects. In summary, at present, surgery, radiotherapy, chemotherapy, targeted therapy, and passive immunotherapy cannot restore or rebuild the body's immune surveillance mechanisms, and the risk of tumor recurrence and metastasis remains a serious concern.

Currently approved tumor immunotherapies primarily focus on stimulating adaptive immune responses via T cell activation. However, these T cell-based immunotherapies have notable limitations. For example, PD-1/PD-L1 inhibitors, when used as monotherapy, benefit only 10% to 25% of patients across nearly all major tumor indications. In "cold tumors" (tumors lacking T cell infiltration) or tumors with non-inflammatory T cell infiltration and immunosuppressive tumor microenvironments, the response rate to adaptive immune checkpoint-targeted immunotherapies is particularly low. This highlights the urgent need for next-generation immunotherapies to improve treatment outcomes.

Currently approved cancer immunotherapies mainly target T cell immune checkpoints, such as PD-1/PD-L1, CTLA-4, and LAG-3. As shown in the table below, although T cell immune checkpoint inhibitors (e.g., PD-1/PD-L1 antibodies) have been clinically applied to many cancer types, including as first-line treatments, their response rates remain low across nearly all major tumor indications.

Tumor response rates for PD-1/PD-L1 inhibitor monotherapy

| | NSCL C | SCL C | CRC | GC | HNSC C | HC C | ESC C | BT C | RC C | OC | CC | UC | STS | DLBC L |
|---|---|---|---|---|---|---|---|---|---|---|---|---|---|---|
| PD -1 | 19-20% | 12-19% | <10 % | 13-14 % | 13-16% | 16-17% | 19-20% | 3-22% | 0.22 | 8-15 % | 0.1 4 | 20-29 % | 5-18 % | 0.45 |
| PD -L1 | 0.14 | 2-10% | | | | | | 0.05 | | 0.1 | | 13-24 % | - | |

Notes: (1) Response rates are based on the latest standards from the U.S. FDA and China NMPA, except for colorectal cancer, gastric cancer, small-cell lung cancer, ovarian cancer, cholangiocarcinoma, and soft tissue sarcoma, which are based on published clinical results. (2) Only clinical outcomes for monotherapy are listed. (3) Adjuvant therapy results are not included. Results may vary across different cancer subtypes or clinical trials. (4) The listed clinical outcomes are derived from general cancer populations (regardless of PD-L1 expression), except for the overall response rate of cervical cancer, which is limited to the PD-L1 positive population with a combined positive score (CPS) ≥1.

Definition: NSCLC refers to non-small cell lung cancer; SCLC refers to small cell lung cancer; CRC refers to colorectal cancer; GC refers to gastric cancer; HNSCC refers to head and neck squamous cell carcinoma; HCC refers to hepatocellular carcinoma; ESCC refers to esophageal squamous cell carcinoma; BDC refers to bile duct cancer; RCC refers to renal cell carcinoma; OC refers to ovarian cancer; CC refers to cervical cancer; UC refers to urothelial carcinoma; STS refers to soft tissue sarcoma; DLBCL refers to diffuse large B-cell lymphoma.

Data source: Frost & Sullivan

The safety and efficacy of other T-cell immunotherapies remain to be improved. Although chimeric antigen receptor T-cell (CAR-T) immunotherapy can induce significant and durable remissions in certain B-cell leukemia, lymphoma, and multiple myeloma (MM) subpopulations, it still faces several limitations, including life-threatening cytokine release syndrome (CRS) and neurotoxicity, extremely high costs, and limited efficacy against solid tumors. Similarly, T-cell engaging antibodies, such as CD3-based bispecific antibodies, also present concerning safety issues, including severe cytokine release syndrome and "off-tumor" toxicity to healthy tissues. To date, intolerable toxicities associated with CAR-T therapies or CD3 bispecific antibodies have led to the termination or suspension of multiple global clinical studies for numerous candidates, including Atara's ATA2271 (autologous mesothelin CAR-T), Amgen's AMG673 (CD3×CD33), AMG427 (CD3×FLT3), AMG701 (CD3×BCMA), Regeneron's odronextamab (CD3×CD20), and Pfizer's elranatamab (CD3×BCMA).

According to Frost & Sullivan, for the treatment of solid tumors, there is currently only one T-cell engaging antibody on the market--tebentafusp--which is approved for the treatment of uveal melanoma (a rare disease), and no other CAR-T therapies are approved anywhere globally for the treatment of solid tumors.

Recent studies indicate that leveraging the innate immune system, as well as the synergy between the innate and adaptive immune systems, can help overcome the limitations of current immunotherapies. To date, there is no globally approved therapy that activates the innate immune system, thereby stimulating the adaptive immune system, and achieves multi-targeted anticancer effects through the coordinated action of both innate and adaptive immunity.

Tumor-directed active immunotherapy has emerged as a revolutionary approach in cancer treatment, aiming to eliminate cancer cells by stimulating and activating the patient's own immune system.

In general, the human immune system can be divided into the innate immune system and the adaptive immune system. The innate immune system serves as the first line of defense, capable of recognizing foreign substances and rapidly initiating a non-specific immune response. The main innate immune cells include macrophages, natural killer (NK) cells, and dendritic cells (DCs). The adaptive immune system, which includes T cells and B cells, acts as the second line of defense and can more efficiently recognize and eliminate specific antigens. The following table compares key adaptive and innate immune cells within the tumor microenvironment:

| Activation Process | Requires antigen presentation | First line of defense, rapid response, no need for antigen presentation | | |
|---|---|---|---|---|
| Key Immune Cell Types | T Cells | Macrophages | Natural Killer (NK) Cells | Dendritic Cells (DCs) |
| Tumor Tissue Distribution | 10%-30% | 20%-50% | 5%-10% | 3%-10% |
| Major Immune Checkpoints | PD-1/PD-L1, CTLA-4, LAG-3, TIM-3, TIGIT | CD47/SIRPα, CD24/Siglec-10, PSGL-1, EP4 | KIR family, CD94-NKG2A, CD24/Siglec-10, TIGIT, EP4 | PD-1/PD-L1, CD47/SIRPα, EP4 |

(continued)

| Activation Process | Requires antigen presentation | First line of defense, rapid response, no need for antigen presentation | | |
|---|---|---|---|---|
| Major Immune Functions | • T cells mediate tumor cell killing through exocytosis of cytotoxic granules (perforin, granzyme) and secretion of anti-tumor cytokines | • Macrophage-mediated phago-cytosis<br>• Recruitment of T cells to the tumor microenvironment<br>•Antigen presentation<br>• Trogocytosis | • NK cells mediate cytolysis through the secretion of perforin and granzyme<br>• Activate T cells, macrophages, and dendritic cells via cytokine release | • Recruitment of T cells to the tumor microenvironment<br>• Antigen presentation |

Note: Tumor tissue distribution refers to the proportion of various immune cells present in different tumor tissues. Source: Frost & Sullivan.

Compared with adaptive immune cells, innate immune cells are more broadly distributed within tumor tissues. In addition to serving as the first line of defense, innate immune cells play a crucial role in activating adaptive immune responses, thereby generating a more complete and effective immune reaction. For example, activated macrophages and dendritic cells can secrete cytokines and chemokines (such as CXCL9 and CXCL10) to recruit T cells into the tumor microenvironment, thereby converting "cold tumors" into "hot tumors"--those infiltrated by T cells and responsive to immunotherapy. Macrophages and dendritic cells further enhance T-cell responses through antigen presentation. Once activated, natural killer (NK) cells promote T-cell differentiation and activation, amplifying T-cell-mediated responses. Therefore, therapies that target the innate immune system to activate adaptive immunity hold significant potential to overcome the current limitations of approved T-cell-based immunotherapies.

In recent years, numerous studies have revealed the potential of targeting innate immunity to overcome the limitations of T cell-based immunotherapies. Innate immune cells are widely distributed within tumor tissues and, once activated, can directly attack cancer cells while also enhancing adaptive immune responses through interactions with T cells. For example, macrophages can be activated through macrophage-targeted immunotherapies, which in turn trigger effective adaptive immune activation. As major antigen-presenting cells, macrophages can release cytokines and chemokines that recruit T cells; thus, macrophage activation enhances T-cell abundance within the tumor microenvironment, converting "cold tumors" into "hot tumors." Other important innate immune cells--such as natural killer (NK) cells and dendritic cells (DCs)--can also enhance T-cell immune responses through various mechanisms. Therapeutic strategies that leverage the synergy between innate and adaptive immunity maximize immunotherapeutic efficacy and hold great potential for achieving potent antitumor activity even in "cold tumors."

Academician Wang Zhenyi, a renowned Chinese hematologic oncologist and the global pioneer of induced differentiation therapy for acute promyelocytic leukemia (APL), proposed as early as the 1980s that combating cancer requires a "shift in thinking." He advocated improving the body's overall immunity and implementing multi-target immune attacks, or "multi-component, multi-target action." His concept includes three key points: (1) The formation and progression of cancer are in dynamic balance with the immune system. As long as immune competence exists and remains sufficiently strong, cancer cells cannot form or will quickly undergo apoptosis or dormancy. Cancer cells both proliferate and die continuously; when the immune-tumor balance slightly shifts toward immunity, tumors cease to grow due to massive cancer cell apoptosis. (2) Tumor cells are highly complex, with numerous "targets" (antigens) on their surface and within. Importantly, cancer cells exhibit genetic instability. Unlike normal cells, which faithfully replicate genetic information during division, cancer cells continually alter their genetic makeup, giving rise to countless heterogeneous subclones. The foundation of antitumor immunity lies in the ability of diverse immune cells and cytokines to act on multiple tumor targets; a single immune cell type or factor (single-handedly) may not be sufficient to eliminate cancer cells. (3) Effective immunotherapy should rely on stimulation, not supplementation-that is, activating not just one immune component but mobilizing the entire immune system ("magnificent army with thousands of men and horses"). Once activated, immune components can adapt dynamically to the evolving genetic profile of tumor cells, ensuring sustained and effective attacks against mutated cancer cells.

Bacterial inoculation therapy for malignant tumors was first developed in the 1890s by American physician Dr. William Coley, and was primarily used in the United States until the 1960s, when it was officially halted by the U.S. FDA. Historically, this treatment approach became known as Coley's Toxin Therapy. One of the most notable characteristics of the Coley regimen was its inconsistent efficacy--therapeutic outcomes varied significantly across patients and tumor types. The primary reason for this inconsistency was the lack of a stable mechanism for cancer cell recognition. Similarly, in China today, therapies that stimulate the immune system through bacterial activation face comparable challenges and therefore have not become mainstream cancer treatments.

Tumor metastasis refers to the process by which primary tumor cells invade or spread to other tissues and subsequently colonize and proliferate. Metastasis remains the leading cause of cancer-related deaths, yet the biological mechanisms underlying this highly complex process are still poorly understood. Currently, there are few, if any, effective broad-spectrum drugs capable of preventing or inhibiting cancer cell dissemination and metastasis. As mentioned earlier, the major first-line cancer therapies--such as small-molecule targeted drugs, immune checkpoint inhibitors, and CAR-T cell therapies--show limited efficacy and are only effective in a small subset of localized (primary) tumors, while being largely ineffective against metastatic lesions.

SUMMARY

The objective of the present disclosure is to provide a pharmaceutical composition, as well as its preparation method and use.

To achieve the above-mentioned objectives and other related objectives, the present disclosure provides a pharmaceutical composition. The pharmaceutical composition includes a first active ingredient, a second active ingredient, and a pharmaceutically acceptable carrier or excipient. The first active ingredient is a microbial agent, including one or more of *Staphylococcus aureus, Bordetella pertussis,* diphtheria toxoid, tetanus toxoid, *Salmonella typhi,* and *Salmonella paratyphi.* The second active ingredient includes polyinosinic acid, polycytidylic acid, and vitamin.

The present disclosure also provides a use of the pharmaceutical composition in the preparation of a product for treating, preventing, or diagnosing a disease.

The disease is selected from cancer, arteriosclerosis, HPV infection, and atrophic gastritis; preferably, the cancer is selected from liver cancer, lung cancer, melanoma, colorectal cancer, gastric cancer, ovarian cancer, cholangiocarcinoma, cervical cancer, pancreatic cancer, etc.

As described above, the pharmaceutical composition PGc biological injection of the present disclosure, as well as its preparation method and use, have the following beneficial effects:

1. The PGc biological injection is an artificial active immunotherapy for tumors. It can "stimulate" the entire immune system, making the therapy of using bacteria to activate the human immune system to kill cancer cells highly stable and reliable. This can significantly save and prolong the lives of cancer patients while improving their quality of life.
2. PGc has extremely high safety with minimal toxic side effects. When patients are injected with PGc, no other side effects have been reported to date, except for immune responses such as mild erythema and swelling at the injection site and transient fever.
3. The PGc biological injection has a low production cost, making it a sustainable cancer treatment option for patients, ensuring that the cancer treatment is accessible to all patients in need.
4. According to statistics from the National Health Education Book on Major Disease Prevention released by the China Association of Actuaries in 2021, the average total cost of cancer treatment ranges from 220,000 to 800,000 RMB per person. In contrast, the PGc biological injection can reduce the treatment cost for patients to within 40,000 RMB.
5. The broad applicability of the PGc biological injection also provides a new and effective treatment option for currently difficult-to-treat conditions such as atherosclerosis, HPV-positive to negative conversion, and atrophic gastritis.

In conclusion, the widespread use of the PGc biological injection in treatment can effectively improve public health and significantly prolong human lifespan.

BRIEF DESCRIPTION OF THE DRAWINGS

FIG. 1 shows the tumor changes in ten animals after administration of different samples of the present disclosure.
FIG. 2 shows the results of the PGc formulation screening supplementatary experiment in Example 2 of the present disclosure.
FIG. 3 shows the results of the PGc formulation optimization experiment in Example 3 of the present disclosure.
FIG. 4 shows the typical lung anatomical images of the NS group and PGc group in Example 4 of the present disclosure.
FIG. 5 shows the experimental design diagram in Example 5 of the present disclosure.
FIG. 6 shows the schematic diagram of the administration sites in Example 5 of the present disclosure.
FIG. 7 shows the experimental results in Example 5 of the present disclosure.

FIG. 8 shows the anatomical results in Example 6 of the present disclosure.

FIG. 9 shows the statistical results in Example 6 of the present disclosure.

FIG. 10 shows the statistical results in Example 7 of the present disclosure.

FIG. 11 shows the tumor anatomical image in Example 8 of the present disclosure.

FIG. 12 shows the tumor anatomical image in Example 9 of the present disclosure.

FIG. 13 shows the tumor anatomical image in Example 10 of the present disclosure.

FIG. 14 shows the tumor anatomical image in Example 11 of the present disclosure.

FIG. 15 shows the tumor anatomical image in Example 12 of the present disclosure.

FIG. 16 shows the results of PGc promoting M1 macrophage activation in Example 14 of the present disclosure.

FIG. 17 shows the results of PGc promoting M1 macrophage infiltration in tumor tissue in Example 14 of the present disclosure.

FIG. 18 shows the results of PGc enhancing T lymphocyte infiltration in tumor tissue in Example 14 of the present disclosure.

FIG. 19 shows the results of PGc inhibiting angiogenesis in tumor tissue in Example 14 of the present disclosure.

FIG. 20 shows the biological process enrichment analysis in Example 14 of the present disclosure, reflecting the level to which PGc activates various immune responses.

FIG. 21 shows the KEGG signaling pathway enrichment analysis in Example 14 of the present disclosure, reflecting the activation level of immune-related signaling pathways after PGc treatment.

FIG. 22 shows the molecular function enrichment analysis of genes in Example 14 of the present disclosure, reflecting the activation level of the immune system at the molecular level after PGc treatment.

FIG. 23 shows the flow cytometry analysis of changes in T cells, NK cells, NKT cells, and B cells in tumor tissue collected under LLC model mice after PGc treatment in Example 14 of the present disclosure.

## DETAILED DESCRIPTION

The present disclosure provides a pharmaceutical composition including a first active ingredient, a second active ingredient, and a pharmaceutically acceptable carrier or excipient. The first active ingredient is a microbial agent including one or more of *Staphylococcus aureus, Bordetella pertussis,* diphtheria toxoid, tetanus toxoid, *Salmonella typhi,* and *Salmonella paratyphi.* The second active ingredient includes polyinosinic acid, polycytidylic acid, and vitamin.

The microbial agent may be a mixed agent consisting of *Staphylococcus aureus, Bordetella pertussis,* diphtheria toxoid, tetanus toxoid, *Salmonella typhi,* and *Salmonella paratyphi;* or it may be an individual agent of any one of the above, meaning the microbial agent may be a *Staphylococcus aureus* agent, a *Bordetella pertussis* agent, a diphtheria toxoid, a tetanus toxoid, a *Salmonella typhi* agent, or a *Salmonella paratyphi* agent.

The microbial agent is an inactivated preparation.

The microbial agent is in a liquid preparation or a solid preparation. In one embodiment, the microbial agent is a liquid preparation. The liquid preparation includes the fermentation broth of the corresponding microorganism, preferably the crude fermentation broth.

The fermentation broth can be obtained commercially or can be prepared independently.

The *Bordetella pertussis* agent, diphtheria toxoid, and tetanus toxoid can be formulated as three separate agents, or as a combination of two agents together with a separate agent, or as a three-component combined agent.

In certain embodiments of the present disclosure, *Staphylococcus aureus* is prepared in accordance with the bacterial solution preparation procedures described in the 1979 edition of the *Requirements for Biological Products,* specifically following the interim procedures for the production and inspection of 05 *Staphylococcus aureus* vaccine.

In certain embodiments of the present disclosure, *Bordetella pertussis* and diphtheria toxin are prepared in accordance with the specifications for "Diphtheria and Pertussis Combined Vaccine, Adsorbed" as described in Part III of the *Pharmacopoeia of the People's Republic of China.*

In certain embodiments of the present disclosure, the diphtheria toxoid may be prepared in accordance with the specifications for the "Diphtheria Vaccine, Adsorbed" as prescribed in Part III of the *Pharmacopoeia of the People's Republic of China.*

In certain embodiments of the present disclosure, the tetanus toxoid may be prepared in accordance with the specifications for the "Tetanus Vaccine, Adsorbed" as prescribed in Part III of the *Pharmacopoeia of the People's Republic of China.*

In certain embodiments of the present disclosure, *Salmonella typhi* may be prepared in accordance with the specifications for the "Typhoid Vaccine" as prescribed in Part III of the *Pharmacopoeia of the People's Republic of China.*

In certain embodiments of the present disclosure, the *Salmonella paratyphi* may be prepared in accordance with the specifications for the "Typhoid and Paratyphoid A and B Combined Vaccine" as prescribed in Part III of the *Pharmacopoeia of the People's Republic of China.*

In certain embodiments of the present disclosure, based on the total volume of the pharmaceutical composition, the concentration of *Staphylococcus aureus* is $2\times10^7$-$3\times10^9$ CFU/mL.

In certain embodiments of the present disclosure, based on the total volume of the pharmaceutical composition, the concentration of *Bordetella pertussis* is $7\times10^7$-$9\times10^9$ CFU/mL.

In certain embodiments of the present disclosure, based on the total volume of the pharmaceutical composition, the concentration of diphtheria toxoid is 1 LF-5 LF/mL.

In certain embodiments of the present disclosure, based on the total volume of the pharmaceutical composition, the concentration of tetanus toxoid is 0.1 LF-5 LF/mL.

In certain embodiments of the present disclosure, based on the total volume of the pharmaceutical composition, the concentration of *Salmonella typhi* is $1.5\times10^6$-$5\times10^8$ CFU/mL.

In certain embodiments of the present disclosure, based on the total volume of the pharmaceutical composition, the concentration of *Salmonella paratyphi* is $1\times 10^6$-$3\times10^8$ CFU/mL.

The *Salmonella paratyphi* is one or more of *Salmonella paratyphi A, Salmonella paratyphi B,* and *Salmonella paratyphi C.*

The *Salmonella typhi* or *Salmonella paratyphi* may be formulated as separate individual agents, or as a three-component formulation consisting of *Salmonella typhi, Salmonella paratyphi A, and Salmonella paratyphi B,* or as a four-component formulation consisting of *Salmonella typhi, Salmonella paratyphi A, Salmonella paratyphi B,* and *Salmonella paratyphi C.*

In certain embodiments of the present disclosure, the *Salmonella paratyphi* includes *Salmonella paratyphi A* and/or *Salmonella paratyphi B.* Based on the total volume of the pharmaceutical composition, the concentration of *Salmonella paratyphi A* is $0.1\times10^7$-$8\times10^7$ CFU/mL, and/or the concentration of *Salmonella paratyphi B* is $0.1\times10^7$-$8\times10^7$ CFU/mL.

The polyinosinic acid and the polycytidylic acid are selected from a polymer formed solely from inosinic acid, a polymer formed solely from cytidylic acid, as well as a copolymer of inosinic acid and cytidylic acid, i.e., poly(I:C). Poly(I:C) is a double-stranded RNA analog, with one strand being poly(I) and the other strand being poly(C). Correspondingly, the second active ingredient is obtained by mixing polyinosinic acid, polycytidylic acid, and the vitamin, or by mixing poly(I:C) and the vitamin.

In certain embodiments of the present disclosure, based on the total volume of the pharmaceutical composition, the concentrations of the polyinosinic acid and the polycytidylic acid are less than 0.5g/100mL, respectively; for example, 0.01g/100mL-0.5g/100mL. For another example, the concentrations of the polyinosinic acid and polycytidylic acid are each selected from any one of the following concentration ranges: *0.01* g/100 mL to 0.05 *g/100* mL, 0.05 g/100 mL to 0.1 g/100 mL, 0.1 g/100 mL to 0.2 g/100 mL, 0.2 g/100 mL to 0.3 g/100 mL, 0.3 g/100 mL to 0.4 g/100 mL, and 0.4 g/100 mL to 0.5 g/100 mL.

In certain embodiments of the present disclosure, when the polyinosinic acid and polycytidylic acid are a polymer formed solely from inosinic acid and a polymer formed solely from cytidylic acid, respectively, the mass ratio of the polyinosinic acid to the polycytidylic acid is 1:0.1 to 1:10. For example, the mass ratio of the polyinosinic acid to the polycytidylic acid may be 1:0.1 to 1:0.5, 1:0.5 to 1:1, 1:1 to 1:2, 1:2 to 1:4, 1:4 to 1:6, 1:6 to 1:8, or 1:8 to 1:10. In a preferred embodiment of the present disclosure, the mass ratio of the polyinosinic acid to the polycytidylic acid is 1:1. Specifically, when the mass ratio of the polyinosinic acid to the polycytidylic acid is 1:1, the parts by weight of the polyinosinic acid and the polycytidylic acid can be 2 parts, 4 parts, 6 parts, 8 parts, or 10 parts, respectively.

The polyinosinic acid and the polycytidylic acid should comply with the standards of the *Pharmacopoeia of the People's Republic of China,* with no specific requirements regarding their degree of polymerization.

The vitamin is Vitamin A. Vitamin A is Vitamin Al and/or Vitamin A2.

In certain embodiments of the present disclosure, based on the total volume of the pharmaceutical composition, the concentration of Vitamin A1 is less than 1g/100mL. For example, the concentration of the vitamin is selected from any one of the following concentration ranges: 0.1 g/100 mL to 0.2 g/100 mL, 0.2 g/100 mL to 0.3 g/100 mL, 0.3 g/100 mL to 0.4 g/100 mL, 0.4 g/100 mL to 0.5 g/100 mL, 0.5 g/100 mL to 0.8 g/100 mL, and 0.8 g/100 mL to 1.0 g/100 mL.

Polyinosinic acid, polycytidylic acid, and Vitamin A synergistically work together to enable easier identification of abnormal cells in the human body (e.g., tumor cells), causing changes to cell membranes of these abnormal cells.

With respect to the pharmaceutically acceptable carrier or excipient, the term "pharmaceutically acceptable" refers to the fact that when the drug is appropriately administered to animals or humans, it will not cause adverse, allergic, or other harmful reactions.

The "pharmaceutically acceptable carrier or excipient" should be compatible with the active ingredient, meaning it can be mixed with the active ingredient without significantly reducing the drug's effectiveness under normal conditions. Specific examples of substances that may serve as pharmaceutically acceptable carriers or excipients include: aaccharides, such as lactose, glucose, and sucrose; starches, such as corn starch and potato starch; cellulose and its derivatives, such as sodium methylcellulose, ethylcellulose, and methylcellulose; astragalus gummifer powder; malt; gelatin; talc; solid lubricants, such as stearic acid and magnesium stearate; calcium sulfate; vegetable oils, such as peanut oil, cottonseed oil, sesame oil, olive oil, corn oil, and cocoa butter; polyols, such as propylene glycol, glycerol, sorbitol, mannitol, and polyethylene glycol; alginic acid; emulsifiers, such as Tween; wetting agents, such as sodium lauryl sulfate; coloring agents; flavoring agents; tableting agents; stabilizers; antioxidants; preservatives; pyrogen-free water; isotonic saline solutions; or phosphate buffer solutions, etc. These substances are used as needed to enhance the stability of the formulation, improve the activity or bioavailability of the active ingredient, or, in the case of oral administration, to provide an acceptable taste or odor.

In certain embodiments of the present disclosure, the pharmaceutically acceptable carrier or excipient includes sodium carboxymethyl cellulose, aluminum stearate, Tween 80, lecithin, soybean oil, dextran, fat emulsion, and water.

The aluminum stearate is aluminum monostearate or aluminum distearate.

In certain embodiments of the present disclosure, based on the total volume of the pharmaceutical composition, the concentration of the sodium carboxymethyl cellulose in the pharmaceutically acceptable carrier or excipient is less than 2g/100mL. Based on the total volume of the pharmaceutical composition, the concentration of the sodium carboxymethyl cellulose is 0.2-2g/100mL. For example, the concentration of the sodium carboxymethyl cellulose is selected from any one of the following concentration ranges: 0.2g/100mL to 0.5g/100mL, 0.5g/100mL to 1.0g/100mL, 1.0g/100mL to 1.5g/100mL, and 1.5g/100mL to 2g/100mL.

Based on the total volume of the pharmaceutical composition, the concentration of the aluminum stearate in the pharmaceutically acceptable carrier or excipient is less than 3g/100mL. Based on the total volume of the pharmaceutical composition, the concentration of the aluminum stearate is 0.3-3g/100mL. For example, the concentration of the aluminum stearate is selected from any one of the following concentration ranges: 0.3 g/100 mL to 0.5 g/100 mL, 0.5 g/100 mL to 1.0 g/100 mL, 1.0 g/100 mL to 1.5 g/100 mL, 1.5 g/100 mL to 2.0 g/100 mL, 2.0 g/100 mL to 2.5 g/100 mL, and 2.5 g/100 mL to 3.0 g/100 mL.

Based on the total volume of the pharmaceutical composition, the concentration of the Tween 80 in the pharmaceutically acceptable carrier or excipient is less than 1mL/100mL. Based on the total volume of the pharmaceutical composition, the concentration of the Tween 80 is 0.1-1mL/100mL. For example, the concentration of the Tween 80 is selected from any one of the following concentration ranges: 0.1mL/100mL to 0.3mL/100mL, 0.3mL /100mL to 0.5mL/100mL, 0.5mL/100mL to 0.7mL/100mL, and 0.7mL/100mL to 1.0mL/100mL.

Based on the total volume of the pharmaceutical composition, the concentration of the lecithin in the pharmaceutically acceptable carrier or excipient is more than 50mg/100mL. Based on the total volume of the pharmaceutical composition, the concentration of the lecithin is 50-2000mg/100mL. For example, the concentration of the lecithin is selected from any one of the following concentration ranges: 50mg/100mL to 100mg/100mL, 100mg/100mL to 300mg/100mL, 300mg/100mL to 500mg/100mL, 500mg/100mL to 1500mg/100mL, and 1500mL to 2000mg/100mL.

Based on the total volume of the pharmaceutical composition, the concentration of the soybean oil in the pharmaceutically acceptable carrier or excipient is less than 15ml/100mL. Based on the total volume of the pharmaceutical composition, the concentration of the soybean oil is 1-15mL/100mL. For example, the concentration of the soybean oil is selected from any one of the following concentration ranges: 1mL/100mL to 3mL/100mL, 3 mL /100mL to 6 mL /100mL, 6 mL /100mL to 9 mL /100mL, 9 mL /100mL to 12 mL /100mL, and 12 mL /100mL to 15 mL /100mL.

Based on the total volume of the pharmaceutical composition, the concentration of the dextran in the pharmaceutically acceptable carrier or excipient is less than 10g/100mL. Based on the total volume of the pharmaceutical composition, the concentration of the dextran is 1-10g/100mL. For example, the concentration of the dextran is selected from any one of the following concentration ranges: 1g/100mL to 2g/100mL, 2g/100mL to 4g/100mL, 4g/100mL to 6g/100mL, 6g/100mL to 8g/100mL, and 8g/100mL to 10g/100mL.

Based on the total volume of the pharmaceutical composition, the concentration of the fat emulsion in the pharmaceutically acceptable carrier or excipient is more than 10ml/100mL. Based on the total volume of the pharmaceutical composition, the concentration of the fat emulsion is 10-80mL/100mL. For example, the concentration of the fat emulsion is selected from any one of the following concentration ranges: 10mL/100mL to 20mL/100mL, 20 mL /100mL to 30mL /100mL, 30mL /100mL to 40mL /100mL, 40 mL /100mL to 50mL /100mL, 50 mL /100mL to 60 mL /100mL, 60 mL /100mL to 70mL /100mL, and 70 mL /100mL to 80 mL /100mL.

The combination of aluminum stearate and lecithin can effectively facilitate the infiltration of effector macrophages and T cells into the tumor microenvironment.

The pharmaceutical composition described herein is not limited by any specific form and can exist in various physical forms, such as solid, liquid, gel, semi-liquid, aerosol, and others.

In certain embodiments of the present disclosure, the pharmaceutical composition is an injection.

The present disclosure also provides a method for preparing the pharmaceutical composition, which includes mixing the first active ingredient, the second active ingredient, and the pharmaceutically acceptable carrier or excipient to obtain the composition.

The present disclosure also provides a use of the pharmaceutical composition in the preparation of a product for treating, preventing, or diagnosing a disease.

In the present disclosure, the term "prevention" or "preventing" refers to prophylactic measures that can result in desired pharmacological and/or physiological effects. The preferred effect refers to an effect that, from a medical perspective, can block or delay the onset of a disease and/or reduce the risk of disease progression or worsening.

In the present disclosure, the term "diagnosis" or "diagnosing" refers to the ability to determine the presence of a particular disease.

In the present disclosure, the term "treatment" or "treating" includes curative or palliative measures that can result in desired pharmacological and/or physiological effects. The preferable effect refers to an effect that, from a medical perspective, can reduce one or more symptoms of a disease or completely eliminate the disease.

The diseases described herein are selected from cancers, arteriosclerosis, HPV infection, and atrophic gastritis.

In the present disclosure, the term "cancer" refers to any medical condition mediated by the growth, proliferation, or metastasis of tumors or malignant cells, encompassing both solid and non-solid tumors such as leukemia. In the present disclosure, the term "tumor" refers to the physical mass of a tumor and/or malignant cells.

The cancers described herein include, but are not limited to, liver cancer, lung cancer, melanoma, colorectal cancer, gastric cancer, ovarian cancer, cholangiocarcinoma, cervical cancer, and pancreatic cancer.

The arteriosclerosis includes atherosclerosis, Mönckeberg's arteriosclerosis, and arteriolosclerosis.

The HPV infection described herein may be high-risk, intermediate-risk, or low-risk types. High-risk types include HPV16, 18, 31, 33, 35, 39, 45, 51, 52, 56, 58, 59, and 68. Intermediate-risk types include HPV26, 53, 66, 73, and 82. Low-risk types include HPV6, 11, 40, 42, 43, 44, 54, 61, 70, 72, 81, and 89.

The present disclosure further provides a method for treating or preventing a disease, including administering to a subject a therapeutically effective amount of the pharmaceutical composition described herein.

The term "subject" includes, but is not limited to, animals, preferably mammals. The mammals include rodents, even-toed ungulates, odd-toed ungulates, lagomorphs, and primates. Examples of mammals include, but are not limited to, humans, non-human primates (e.g., monkeys), mice, pigs, cattle, goats, rabbits, rats, guinea pigs, hamsters, horses, monkeys, and sheep, or other non-human mammals. Non-mammals include, for example, non-mammalian vertebrates such as birds (e.g., chickens or ducks) or fish, and non-mammalian invertebrates. The subject may be a human, for example, a patient with immunodeficiency or cancer.

The terms "treatment" or "therapy" of a condition include preventing or alleviating the condition, delaying the onset or slowing progression of the condition, reducing the risk of developing the condition, preventing or delaying the development of symptoms associated with the condition, reducing or eliminating symptoms associated with the condition, partially or completely reversing the condition, curing the condition, or any combination thereof. For cancer, "treatment" or "therapy" may refer to inhibiting or slowing the growth, proliferation, or metastasis of tumors or malignant cells, or any combination thereof. For tumors, "treatment" or "therapy" includes eliminating all or part of the tumor, inhibiting or slowing tumor growth and metastasis, preventing or delaying tumor progression, or any combination thereof.

The cancers include, but are not limited to: lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, and thymic carcinoma; as well as hematologic malignancies, such as leukemia, lymphoma, multiple myeloma, mycosis fungoides, Merkel cell carcinoma, and other malignant hematologic disorders, including classical Hodgkin lymphoma (CHL), primary mediastinal large B-cell lymphoma, T-cell/histiocyte-rich large B-cell lymphoma, EBV-positive and EBV-negative post-transplant lymphoproliferative disorder (PTLD), EBV-associated diffuse large B-cell lymphoma (DLBCL), plasmablastic lymphoma, nasopharyngeal carcinoma, and HHV8-associated primary effusion lymphoma.

In the present disclosure, a "therapeutically effective amount" or "effective dose" refers to an amount or concentration of a drug that is sufficient to effectively treat a disease or condition. For example, with respect to the use of the pharmaceutical composition in the present disclosure, a therapeutically effective amount refers to a dose or concentration at which the pharmaceutical composition can eliminate all or part of the tumor, inhibit or slow tumor growth, inhibit the growth or proliferation of cells mediating the cancerous state, suppress tumor cell metastasis, alleviate any symptoms or markers associated with the tumor or cancerous state, prevent or delay the progression of the tumor or cancerous state, or any combination thereof.

Specifically, when administered to a subject, the dosage may vary depending on the patient's age, body weight, disease characteristics and severity, and route of administration. Dosage can also be guided by results from animal studies and other relevant considerations. The total administered dose should not exceed a defined safe range.

In certain embodiments, the methods described herein may further include coadministration with other compounds or combination with other existing cancer therapies.

Other cancer therapies may include, but are not limited to: surgery, radiotherapy, chemotherapy, toxin therapy, immunotherapy, cryotherapy, cancer vaccines (e.g., HPV vaccine, hepatitis B vaccine, Oncophage, Provenge), and gene therapy, as well as any combination thereof. Immunotherapy includes, but is not limited to, adoptive cell therapy, derivation of stem cells and/or dendritic cells, transfusion, perfusion, and/or other approaches, including, but not limited to, cryoablation of tumor.

The present disclosure is further illustrated through specific examples; those skilled in the art can easily understand other advantages and effects of the present disclosure based on the content disclosed in this specification.

The present disclosure may also be implemented or applied through various other specific embodiments. The details disclosed herein may be modified or altered from different perspectives or for different applications without departing from the spirit of the present disclosure.

Before further describing the specific embodiments of the present disclosure, it should be understood that the scope of the present disclosure is not limited to the specific examples provided below. It should also be understood that the term used in the examples is intended to describe particular embodiments and not to limit the scope of the present disclosure. Unless otherwise explicitly stated, the singular forms "a", "an", and "the" include the plural forms.

When numerical ranges are provided in the examples, it should be understood that any value within the range, including the two endpoints, may be selected, unless otherwise specified. Unless otherwise defined, all technical and scientific terms used herein have the same meaning as commonly understood by those skilled in the art. Apart from the specific methods, devices, and materials described in the examples, those skilled in the art may use any similar or equivalent methods, devices, and materials known in the prior art to achieve the same results as described in the embodiments of the present disclosure.

**Example 1: PG Component Screening Experiment**

1Experiment Design

PGa: Did not contain PI (polyinosinic acid) + PC (polycytidylic acid); other components were consistent with those of PGb, PGc, and PGd.

PGb: Did not contain lecithin; other components were consistent with those of PGa, PGc, and PGd.

PGd: Did not contain Va (Vitamin A); other components were consistent with those of PGa, PGb, and PGc.

The following table showed the formulation details for PGa, PGb, PGc, and PGd:

| Sample Name | Inactivated Bacterial Stock Solution | Other Active Pharmaceutical Ingredients | Excipients |
|---|---|---|---|
| PGa | Inactivated *Staphylococcus aureus* stock solution (the final concentration: $4 \times 10^8$ CFU/mL); Inactivated DPT (Diphtheria Tetanus Pertussis vaccine) stock solution (the final concentration of *Bordetella pertussis:* $5.4 \times 10^8$ CFU/mL, the final concentration of diphtheria toxoid: 1.20 LF/mL, the final concentration of tetanus toxoid: 3.3 LF/mL); Inactivated *Salmonella typhi* and *Salmonella paratyphi* stock solution (the final concentration of *Salmonella typhi:* $2.1 \times 10^7$ CFU/mL, the final concentration of *Salmonella paratyphi A:* $1.05 \times 10^7$ CFU/mL, the final concentration of *Salmonella paratyphi B:* $1.05 \times 10^7$ CFU/mL) | Vitamin A2 0.5g/100mL | Sodium carboxymethyl cellulose 0.8g/100mL, Aluminum monostearate 1.3g/100mL, Tween 80 0.68mL/100 mL, Lecithin 0.9g/100mL, Soybean oil 10mL/100mL , Dextran 5.6g/100mL, Fat emulsion 38mL/100mL , Water for injection 12.5mL/100 mL |
| PGb | | Polyinosinic acid + Polycytidylic acid: 0.1g/100mL each; Vitamin A2: 0.5g/100mL | Sodium carboxymethyl cellulose: 0.8g/100mL; Aluminum monostearate: 1.3g/100mL; Tween 80: 0.68mL/100 mL; Soybean oil: 10mL/100mL ; Dextran: 5.6g/100mL; Fat emulsion: 38mL/100mL ; Water for injection: 12.5 mL/100mL |
| PGc | | Polyinosinic acid + Polycytidylic acid: 0.1g/100mL each; Vitamin A2: 0.5g/100mL | Sodium carboxymethyl cellulose: 0.8 g/100mL; Aluminum |

(continued)

| Sample Name | Inactivated Bacterial Stock Solution | Other Active Pharmaceutical Ingredients | Excipients |
|---|---|---|---|
|  |  |  | monostearate: 1.3 g/100mL; Tween 80: 0.68 mL/100mL; Lecithin: 0.9 g/100mL; Soybean oil: 10 mL/100mL; Dextran: 5.6 g/100mL; Fat emulsion: 38 mL/100mL; Water for injection: 12.5 mL/100mL |
| PGd |  | Polyinosinic acid + Polycytidylic acid: 0.1g/100mL each; No vitamin A | Sodium carboxymethyl cellulose: 0.8 g/100mL; Aluminum monostearate: 1.3 g/100mL; Tween 80: 0.68 mL/100mL; Lecithin: 0.9 g/100mL; Soybean oil: 10 mL/100mL; Dextran: 5.6 g/100mL; Fat emulsion: 38 mL/100mL; Water for injection: 12.5 mL/100mL |

| PG Formulation Screening Experiment Protocol | | | | | |
|---|---|---|---|---|---|
| Group | Sample | Dosing Schedule | Dose | Administration Site | Termination |
| Negative Control Group | Normal saline (NS) | First dosing on day 1 after tumor cell inoculation, sc03q3d | 0.1mL/mouse/dose | Subcutaneous injection around and near the tumor site | Tumors were excised and weighed on day 10 after tumor cell inoculation |
| Positive Control Group | Cyclophosphamide (CTX) | First dosing on day 1 after tumor cell inoculation, ip◊7qd | 30mg/kg/dose | Intraperitoneal injection | |
| PGa Group | PGa | First dosing on day 1 after tumor cell inoculation, sc03q3d | 0.1mL/mouse/dose | Subcutaneous injection around and near the tumor site | |
| PGb Group | PGb | First dosing on day 1 after tumor cell inoculation, sc03q3d | 0.1mL/mouse/dose | Subcutaneous injection around and near the tumor site | |
| PGc Group | PGc | First dosing on day 1 after tumor cell inoculation, sc03q3d | 0.1mL/mouse/dose | Subcutaneous injection around and near the tumor site | |
| PGd Group | PGd | First dosing on day 1 after tumor cell inoculation, sc03q3d | 0.1mL/mouse/dose | Subcutaneous injection around and near the tumor site | |
| Note: Each group consisted of 10 C57/BL/6 mice, 9 weeks old. | | | | | |

2. **Main** Experiment **Procedure**

2.1 LLC Cell Culture

The LLC cells were cultured in DMEM medium containing 10% high-quality fetal bovine serum, in a cell incubator with 5% $CO_2$ at 37°C.

2.2 Subcutaneous Tumor Formation

The LLC cells (tumor source) in the logarithmic growth phase were collected, and 0.1 mL of cell suspension (containing approximately $1\times10^6$ cells) was inoculated subcutaneously into the axillary region of the corresponding C57/BL/6 mice.

2.3 Random Grouping

On day 1 after tumor cell inoculation, all mice were randomly grouped before drug administration.

2.4 Animal Dosing

Dosing was performed according to the design of the pharmacological study protocol.

2.5 Tumor Volume Measurement

On day 7 and day 9 after tumor cell inoculation, the length (a) and width (b) of the tumor in each group of animals were measured externally. The tumor volume was estimated using the formula: $V = 0.52 \times a \times b^2$.

2.6 Termination of the Test

On day 10 after tumor cell inoculation, the experiment was terminated, and tumors were excised and weighed from each group of animals.

**3 Test Results and Analysis**

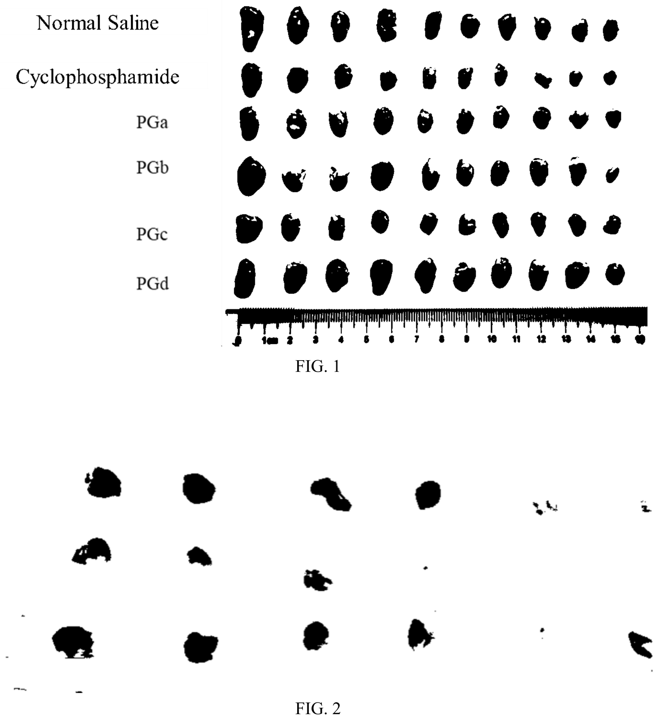

The tumor image was shown in FIG. 1.

Statistical analysis and tumor inhibition rate calculation were performed as follows: Data were statistically analyzed using GraphPad Prism software. The tumor inhibition rate was calculated based on tumor size or tumor weight using the following formula: Tumor Inhibition Rate (%) = [(Average tumor volume or tumor weight of control group - Average tumor volume or tumor weight of treatment group) / Average tumor volume or tumor weight of control group] $\times$ 100%.

| Statistical analysis of tumor volume and calculation of tumor inhibition rate on day 7 after tumor cell inoculation | | | | | |
|---|---|---|---|---|---|
| **Group** | **Sample** | **Number of Animals (n) Start/End** | **Tumor Volume (mm$^3$) X** | **Statistical Analysis** | **Tumor Inhibition Rate** |
| Negative Control Group | Normal saline (NS) | 10/10 | 120.84 | / | / |
| Positive Control Group | Cyclophosphamide (CTX) | 10/10 | 80.40 | P=0.154* | 33% |
| PGa Group | PGa | 10/10 | 99.20 | P=0.437* | 18% |
| PGb Group | PGb | 10/10 | 103.32 | P=0.685* | 15% |
| PGc Group | PGc | 10/10 | 35.67 | P=0.003* | 70% |
| PGd Group | PGd | 10/10 | 51.45 | P=0.022* | 57% |
| Note: *Comparison between the treatment group and the negative control group. | | | | | |

| Statistical analysis of tumor volume and calculation of tumor inhibition rate on day 9 after tumor cell inoculation | | | | | |
|---|---|---|---|---|---|
| **Group** | **Sample** | **Number of Animals (n) Start/End** | **Tumor Volume (mm$^3$) X** | **Statistical Analysis** | **Tumor Inhibition Rate** |
| Negative Control Group | Normal Saline (NS) | 10/10 | 213.88 | / | / |
| Positive Control Group | Cyclophosphamide (CTX) | 10/10 | 112.89 | P=0.023* | 47% |
| PGa Group | PGa | 10/10 | 165.30 | P=0.277* | 23% |
| PGb Group | PGb | 10/10 | 131.89 | P=0.098* | 38% |
| PGc Group | PGc | 10/10 | 103.83 | P=0.027* | 51% |
| PGd Group | PGd | 10/10 | 131.95 | P=0.109* | 38% |
| Note: *Comparison between the treatment group and the negative control group. | | | | | |

| Statistical analysis of tumor volume and calculation of tumor inhibition rate on day 10 after tumor cell inoculation | | | | | |
|---|---|---|---|---|---|
| Group | Sample | Number of Animals (n) Start/End | Tumor Weight (g) X | Statistical Analysis | Tumor Inhibition Rate |
| Negative Control Group | Normal saline (NS) | 10/10 | 0.23 | / | / |
| Positive Control Group | Cyclophosphamide (CTX) | 10/10 | 0.13 | P= 0.031* | 45% |
| PGa Group | PGa | 10/10 | 0.19 | P= 0.217* | 24% |
| PGb Group | PGb | 10/10 | 0.17 | P= 0.570* | -13% |
| PGc Group | PGc | 10/10 | 0.18 | P= 0.058* | 38% |
| PGd Group | PGd | 10/10 | 0.19 | P= 0.779* | -5% |
| Note: *Comparison between the treatment group and the negative control group. | | | | | |

Conclusions:

1 Under the given effective formulation PGc, the new formulation PGa, which was formed by removing the ingredients PI and PC, showed no significant tumor inhibition effect.
2 Under the given effective formulation PGc, the new formulation PGd, which was formed by removing the ingredient Va, showed no tumor inhibition effect.
3 Under the given effective formulation PGc, the new formulation PGb, which was formed by removing the excipient lecithin, showed no tumor inhibition effect.
4 Conclusion: Only under the given condition of the inactivated bacterial stock solution and the ingredients and excipients in the PGc formulation, the coordination and systemic action between the components of PGc can achieve an effective and optimal therapeutic effect.

**Example 2: PGc Component Screening Supplementary Experiment**

**1 Objective** of **the Experiment**

To evaluate the anti-tumor efficacy of the PGc formulation with the simultaneous removal of components PI+PC and Vitamin A.

**2 Test Samples**

2.1 Names: PGc, PGc-PW

The formulation of PGc was the same as that used in Example 1. The formulation of PGc-PW was the same as that of PGc, except that Vitamin A, polyinosinic acid (pI), and polycytidylic acid (PC) were excluded.

2.2 Provided by: CMC

**3 Experimental Materials**

3.1 Negative Control and Vehicle: Sterile normal saline.

**4 Tumor Source**

4.1 Mouse hepatocellular carcinoma cell line H22, provided by Charles River.

**5 Experimental Animals**

5.1 Source: C57/BL/6 mice, provided by Beijing Charles River Laboratory Animal Technology Co., Ltd.

5.2 Age: 9 weeks old

5.3 Gender: Female

5.4 Number of Animals: PGc sample group (2 groups), negative control group (1 group); each group includes 6 mice.

6 **Dosing Regimen and Dose Setting**

7 **Main Experimental Procedures**

7.1 Randomization

| Supplementary Efficacy Study Protocol B/C for Active Component Screening under the H22 tumor model (TY004) -- February 17 | | | | | | | | | | | | | | | |
|---|---|---|---|---|---|---|---|---|---|---|---|---|---|---|---|
| | | | | Tumor Implantation | | Dosing 1 | | | Dosing 2 | | | Dosing 3 | | | Dissection |
| Group | Contro 1 | Number of Animals | Administration Route | Feb 15 | Feb 16 | Feb 17 | Feb 18 | Feb 19 | Feb 20 | Feb 21 | Feb 22 | Feb 23 | Feb 24 | Feb 25 | Feb 26 |
| A | NaCl | 6 | Subcutaneous | Implantation of H22 | Shipment | Ns 0.1 | | | Ns 0.1 | - | | Ns 0.1 | | | Tumor Weighing |
| B | PGc | 6 | Subcutaneous | Implantation of H22 | Shipment | PGc 0.1 | | | PGc 0.1 | - | | PGc 0.1 | | | Tumor Weighing |
| C | PGc-PW | 6 | Subcutaneous | Implantation of H22 | Shipment | PGc-PW 0.1 | | | PGc-PW 0.1 | - | | PGc-PW 0.1 | | | Tumor Weighing |

7.2 Tumor cells in the logarithmic growth phase were harvested and subcutaneously inoculated into the axillary region of the corresponding mice at 0.1 mL per mouse (approximately $2 \times 10^6$ cells). The inoculation was performed by Charles River at its Beijing laboratory. After tumor establishment, the animals were shipped to the Hangzhou laboratory.

7.3 Re-randomization

7.4 Dosing was carried out according to the experimental design. Before each administration, tumor dimensions were measured using a vernier caliper, and tumor volume was calculated.

8 Experimental Result Analysis: The tumor inhibition rate was assessed based on tumor volume changes during the study or determined via the following method: at the end of the experiment, animals in each group were euthanized, tumors were excised and weighed, and the tumor inhibition rate (TIR) was calculated using the following formula: Tumor Inhibition Rate (%)=[(Average Tumor Weight of Control Groug- Average Tumor Weight of Treatment Group)]/ Average Tumor Weight of Control Group $\times$100%.

**9 Experimental** Results

The tumor image was shown in FIG. 2.
Tumor Dissection Data
Weight

Unit: grams (g)

| Mouse ID | A | B | C |
|---|---|---|---|
| 1 | 0.45 | 0.28 | 0.41 |
| 2 | 0.42 | 0.23 | 0.31 |
| 3 | 0.29 | 0.16 | 0.3 |
| 4 | 0.23 | 0.14 | 0.28 |
| 5 | 0.27 | 0.04 | 0.18 |
| 6 | 0.14 | 0.04 | 0.19 |
| Total | 1.8 | 0.89 | 1.67 |
| **TIR** | | **50.60%** | **7.20%** |

Conclusion:

Under conditions where the original PGc formulation demonstrated antitumor efficacy, the newly prepared formulation, lacking the active components PI+PC and vitamin A, exhibited negligible or no tumor-inhibitory activity.

**Example 3: PGc Formulation Optimization Experiment under the Orthotopic Tumor-Bearing Model of H22 Hepatocellular Carcinoma in Mice**

**1 Objective of the Experiment**

a) Evaluation of the efficacy of the PGc formula with Poly (I:C) replacing PI+PC.
b) Evaluation of the efficacy of the PGc formula with varying amounts of PI+PC.

**2 Test Samples**

2.1 Formulation of the Samples:

The formulation used in this experiment differed from that in Example 1 only in the mass ratio of polyinosinic acid (PI) to polycytidylic acid (PC). The other components and their proportions remained unchanged. The specific groups were as follows:

B6: PI+PC ratio 8:8
C6: PI+PC ratio 4:4
D6: PI+PC ratio 8:1
E6: PI+PC ratio 4:1
F6: Only Poly (I:C)
G6: PI+PC ratio 8:8 without fat emulsion added.

2.2 Provider of Samples: CMC

### 3 Experimental Materials

3.1 Negative Control and Vehicle: Sterile normal saline.

### 4 Tumor Source

4.1 Mouse hepatocellular carcinoma cell line H22, provided by Charles River.

### 5 Experimental Animals

5.1 Source: C57/BL/6 mice, provided by Beijing Charles River Laboratory Animal Technology Co., Ltd.

5.2 Age: 9 weeks old.

5.3 Gender: Female.

5.4 Number of Animals: PGc sample group (6 groups), negative control group (1 group); each group includes 6 mice.

### 6 Dosing Regimen and Dose Setting

The same as described in Example 2.

### 7 Main Experimental Procedures

7.1 Randomization

7.2 Tumor cells in the logarithmic growth phase were inoculated subcutaneously into the axilla of the corresponding mice at a dosage of 0.1 mL per mouse (approximately $2 \times 10^6$ cells). The inoculation was carried out by Charles River at its Beijing laboratory. After tumor formation, the animals are shipped to the Hangzhou laboratory.

7.3 Re-randomization

7.4 Dosing was carried out according to the experimental design. Before each administration, tumor dimensions were measured using a vernier caliper, and tumor volume was calculated.

8 Experimental Result Analysis: The tumor inhibition rate was assessed based on tumor volume changes during the study or determined via the following method: at the end of the experiment, animals in each group were euthanized, tumors were excised and weighed, and the tumor inhibition rate (TIR) was calculated using the following formula: Tumor Inhibition Rate (%)=[(Average Tumor Weight of Control Group- Average Tumor Weigh of Treatment Group)]/ Average Tumor Weight of Control Group $\times 100\%$.

### 9 Experimental Results

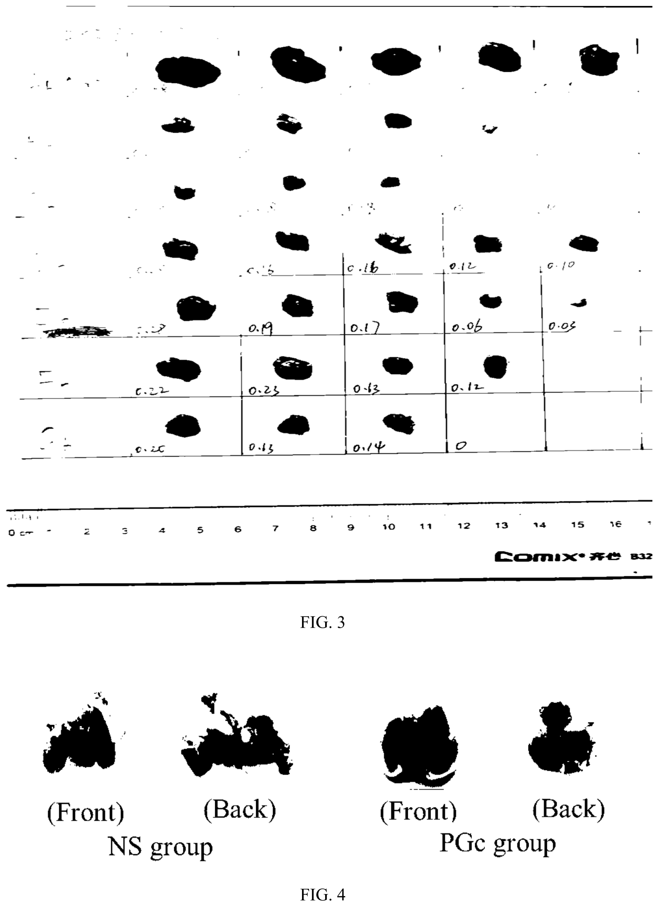

The tumor results were shown in FIG. 3.

| 20230327 | | | | H22 Tumor Inhibition Rate | | |
|---|---|---|---|---|---|---|
| A6 | B6 | C6 | D6 | E6 | F6 | G6 |
| 0.68 | 0.12 | 0.10 | 0.25 | 0.28 | 0.22 | 0.20 |
| 0.64 | 0.12 | 0.08 | 0.16 | 0.19 | 0.23 | 0.13 |
| 0.41 | 0.07 | 0.08 | 0.16 | 0.17 | 0.13 | 0.14 |
| 0.42 | 0.03 | 0.00 | 0.12 | 0.06 | 0.12 | 0.00 |
| 0.39 | 0.00 | 0.00 | 0.10 | 0.03 | | |
| 2.54 | 0.34 | 0.26 | 0.79 | 0.73 | 0.88 | 0.59 |
| TIR | 86.6% | 89.8% | 68.9% | 71.3% | 65.6% | 76.9% |

Conclusions:

1. The PGc biological injection showed a significant inhibitory effect on the growth of liver cancer. The efficacy of the PI+PC combinations, whether in equal or unequal amounts, was superior to Poly (I:C). PI+PC (8:8) with equal amount of PI and PC exhibited significantly better efficacy than Poly (I:C).
2. The optimal group was the PI+PC (4:4).

**Example 4: Evaluation of the Anti-Tumor Efficacy of the PGc Formulation under the B16 Melanoma Mouse Lung Metastasis Model via Tail Vein Injection**

**1 Experimental Design**

| Efficacy Study of the PGc Formulation under the B16 Melanoma Mouse Lung Metastasis Model | | | | | |
|---|---|---|---|---|---|
| Group | Sample | Dosing Schedule | Dose | Administratio n Site | Termination |
| Negative Control Group | Normal saline (NS) | First dosing on day 3 after tumor cell inoculation, sc05 q3d | 0.1mL/mouse/ dose | Subcutaneous, thoracoabdomi nal region | On day 20 after tumor cell in-oculation, the study was ter-minated, the mice were eu-thanized, and the lungs were excised for observations. |
| PGc Group (same formulation as described in Example 1) | PGc | First dosing on day 3 after tumor cell inoculation, sc05 q3d | 0.1mL/mouse/ dose | Subcutaneous, thoracoabdomi nal region | |
| Note: Each group consisted of 6 C57/BL/6 mice, 8 weeks old. | | | | | |

**2. Main Procedures**

2.1 B16 Cell Culture

The B16 cells were cultured in DMEM medium containing 10% high-quality fetal bovine serum (FBS) and maintained in a cell incubator with 5% $CO_2$ at 37°C.

2.2 Tail Vein Inoculation

The B16 cells (tumor source) in the logarithmic growth phase were collected, and 0.1 mL of cell suspension

(approximately $2 \times 10^5$ cells) was inoculated into the tail vein of the corresponding mice.

2.3 Randomization

On day 3 after tumor cell inoculation, all mice were randomly grouped before drug administration.

2.4 Animal Dosing

Dosing was performed according to the design of the pharmacological study protocol.

2.5 Termination of the Study

On Day 20 after tumor cell inoculation, the experiment was terminated. All mice were euthanized, and the lungs were excised for observation. The presence or absence of B16 pulmonary metastatic lesions was recorded, and the number of metastatic lesions with a diameter $\geq$ 2 mm was recorded.

**3 Results and Analysis**

Typical lung anatomical images of the NS group and the PGc group were shown in FIG. 4. Statistical analysis was performed as follows: GraphPad Prism software was used for statistical analysis of the data.

| Statistical analysis of tumor weight and calculation of tumor inhibition rate on day 10 after tumor cell inoculation | | | | | |
|---|---|---|---|---|---|
| Group | Sample | Number of Animals (n) Start/End | Number of animals without pulmonary metastatic lesions | Number of pulmonary metastatic lesions with a diameter $\geq$ 2 mm | Statistical Analysis |
| Negative Control Group | Normal saline (NS) | 6/6 | 0 | 13 | / |
| PGc Group | PGc | 6/6 | 2 | 3 | P= 0.037 * |
| Note: *Comparison of the number of metastatic lesions with a diameter $\geq$ 2 mm between the treatment group and the negative control group. | | | | | |

Conclusion: The PGc biological injection exhibited a statistically significant inhibitory effect on melanoma metastasis and dissemination. This represents a major non-clinical research breakthrough of considerable significance in the development of tumor therapies.

**Example 5: Efficacy Study of PGc under the PBMC Mouse Model**

This study was conducted by Shanghai Dialbio Biotech Co., Ltd.

1. Objective of the Study

This study aimed to evaluate the antitumor activity of the test article PGc administered subcutaneously under a PBMC-humanized mouse model of non-small cell lung cancer (NSCLC) and to compare the efficacy of PGc with that of an anti-human PD-1 antibody (hereinafter referred to as anti-hPD-1) administered via intraperitoneal injection. The test article PGc was provided by PuGong Biotech (Hangzhou) Co., Ltd. (hereinafter referred to as "PuGong Biotech"), while the anti-human PD-1 antibody was commercially purchased.

2. Experiment Materials

2.1 Test Articles

| Test Article | Appearance | Storage Condition | Concentration (mg/mL) | Batch Number | Provider |
|---|---|---|---|---|---|
| PGc (same formulation as described in Example 1) | Emulsion for injection | 4°C | N/A | 20220608 | PuGong Biotech |

2.2 Vehicle

| Test Articles | Vehicle |
|---|---|
| Normal saline | Normal saline |
| PGc | Not applicable |
| anti-hPD-1 | DPBS |

**2.3 Other Reagents or Materials**

| Materials | Brand / Supplier | Catalog Number | Batch Number |
|---|---|---|---|
| anti-hPD-1 | Biointron | B2014 | 20220718Z003 |
| PBMC | Milecell Biotechnologies | PB100C | Donor1: A10s085125 Donor2: P121030702C |
| Sodium chloride injection | Double-Crane Pharmaceutical | H41023384 | 2111283A |
| RPMI 1640 Medium | CORNING | 10-040-cv | 26321010 |
| Fetal Bovine Serum (FBS) | Gibco | 10099-141C | 2366517CP |
| Penicillin-Streptomycin (P/S) | CORNING | 30-002-CI | 30002362 |
| DPBS | TBDscience | DPB2004Y | 20210411 |
| 0.25% Trypsin | GIBCO | 25200-072 | 2276876 |

**2.4 Instruments**

| Instruments | Brand / Manufacturer | Model |
|---|---|---|
| Centrifuge | eppendorf | 5702 |
| Inverted Microscope | Shanghai Teelen | DXY-1 |
| Biological Safety Cabinet | Heal Force | HFsafe-1500LC(A2) |
| $CO_2$ Incubator | Thermo | 371 |
| Water Bath | Shanghai YIHENG Technical | HWS-24 |
| Electronic Pipette | CAPP | PA-100 |
| Manual Pipette (1mL) | eppendorf | L15867K |
| Manual Pipette (100μL) | eppendorf | K54364K |
| Manual Pipette (20μL) | eppendorf | O46951J |
| Cell Counter | Countstar | S2 |

(continued)

| Instruments | Brand / Manufacturer | Model |
|---|---|---|
| Balance | Ohaus | NVE602ZH |
| Vernier Caliper | Mitutoyo (Japan) | CD-15AX |

## 3. Experimental Animals

### 3.1 Animal Information

| | |
|---|---|
| Species and Strain: | NCG mice |
| Number of Animals (n): | 20 |
| Gender and Age: | Female, 6 weeks old |
| Supplier: | GemPharmatech |
| Acclimation Period: | 3-7 days |
| Animal Facility: | SPF-grade room |
| Ambient Temperature: | 20 - 26°C |
| Indoor Relative Humidity: | 40 - 70% |
| Lighting: | Fluorescent lighting, 12 hours light (08:00-20:00) and 12 hours dark cycle |
| Animal Housing: | 2-5 mice per cage |
| Food: | Ad libitum access to standard chow |
| Water: | Autoclaved drinking water provided ad libitum |

### 3.2 Animal Receipt, Health Assessment, and Acclimation Period

Upon arrival at the animal facility, the animals were received and quarantined by animal facility staff. The mice underwent a 7-day quarantine and acclimation period. Prior to PBMC cell inoculation, the animals were inspected by the study personnel. The assessment included evaluation of general appearance, limbs, orifices, and observation for abnormal behaviors at rest or during movement, in order to exclude animals in poor health.

### 3.3 Cage and Animal Identification

Each mouse was assigned a unique identification number. Before group allocation, cage cards were labeled with the project number, species/strain, gender, cage number, and animal ID. After randomization, cage cards were updated to include the experimental group information, in addition to the above information. Grouping information was documented in the study records.

## 4. Experimental Methods and Procedures

### 4.1 Study Design

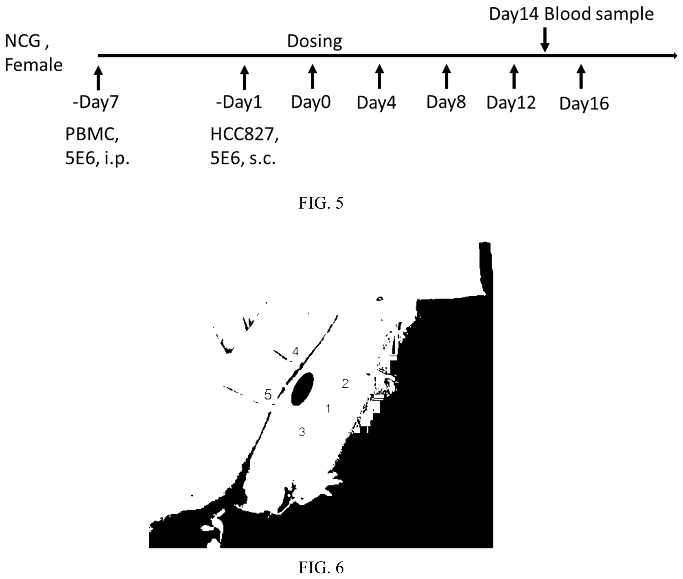

As shown in FIG. 5, on -Day 7, mice were intraperitoneally (i.p.) inoculated with PBMCs at $5 \times 10^6$ cells per mouse. On -Day 1, HCC827 cells (ATCC: CRL-2868) were inoculated subcutaneously at $5 \times 10^6$ cells per mouse. Normal Saline, anti-hPD-1, and PGc were administered on Day 0, Day 4, Day 8, and Day 12, with blood collection on Day 14.
The table below summarizes the grouping and dosing schedule. Groups of 3+3 or 4+4 mice were derived from two donors, with 3 or 4 mice per donor, respectively. Administration routes of Normal Saline Group: Subcutaneous injection (same as PGc group), 5 total administrations during the study. Administration routes of anti-hPD-1 Group: Intraperitoneal injection (i.p.), 5 total administrations during the study. Administration routes of PGc Group (same formulation as Example 1): Subcutaneous injection, 5 total administrations during the study. Administration Sites were shown in FIG. 6, and positions ①,②,③,④, and ⑤ indicated the sequence and locations of administration.

| Group | Number of Mice (n) | Test Articles | Dose | Administration Route | Dosing Schedule |
|---|---|---|---|---|---|
| 1 | 3+3 | normal saline | 100 μL | Subcutaneous | Q4D × 5 |
| 2 | 3+3 | anti-hPD-1 | 5 mg/kg | i.p. | Q4D × 5 |
| 3 | 4+4 | PGc | 100 μL | Subcutaneous | Q4D × 5 |

4.2 Cell Culture and Inoculation

Preparation of two PBMC cell suspensions from two donors: Frozen cell cryopreservation tubes were taken out from liquid nitrogen and thawed in a 37 °C water bath. Cells were centrifuged, the supernatant was removed, and the PBMC cells were resuspended in DPBS. The final PBMC cell concentration was adjusted to $5 \times 10^7$ cells/mL. On October 11, 2022, the PBMC suspensions were intraperitoneally (i.p.) inoculated into mice at a volume of 100 μL per mouse, with 10 mice per donor.

The human lung cancer cell line HCC827 (ATCC, CRL-2868) was cultured in RPMI-1640 supplemented with 10% FBS and 0.1% P/S under 5% $CO_2$ at 37°C. Experiments were conducted before the cells were passaged for 10 generations. Cells were centrifuged, supernatant was removed, and the HCC827 cells were resuspended in DPBS. The final HCC827 cell concentration was adjusted to $5 \times 10^7$ cells/mL. On October 17, 2022, HCC827 cells were subcutaneously (s.c.) inoculated into the right axilla of each mouse at a volume of 100 μL per mouse.

**4.3 Animal Grouping**

1) PBMCs from two donors were inoculated into 20 NCG mice, with 10 mice per donor, designated as Donor 1 group and Donor 2 group. The 20 mice were randomly assigned within each donor group to receive PBMC inoculation.

2) Subsequently, the 10 mice in each donor group were inoculated with HCC827 cells.

3) Prior to dosing, the 10 mice in each donor group were randomly subdivided into three subgroups, with 3 or 4 mice per subgroup (with 4 mice in the PGc subgroup), resulting in a total of six subgroups, namely: Donor1-1, Donor1-2, Donor1-3, Donor2-1, Donor2-2, Donor2-3.

4) Mice were administered according to the following schedule: Normal Saline Group (Donor1-1 and Donor2-1); anti-hPD-1 Group (Donor1-2 and Donor2-2); and PGc Group (Donor1-3 and Donor2-3).

**4.4 Data Collection**

Cage-Side Observation: Animals were observed daily on weekdays for general appearance and behavior, from the time of cell inoculation until the end of the study.

PBMC Donor 1 Model: in normal saline group (i.e., Donor1-1), mice with ear tags of 724, 725, and 727 exhibited hunched posture on Day 24, Day 20, and Day 20, respectively; in anti-hPD-1 group (i.e., Donor1-2), mouse with ear tag of 730 exhibited hunched posture on Day 20 and died on Day 24; in PGc group (i.e., Donor1-3), mice with ear tags of 721, 723, and 726 exhibited hunched posture on Day 20, and mouse with ear tag of 731 died on Day 14.

Additionally, in the PBMC Donor 2 model: in normal saline group (i.e., Donor2-1), mice with ear tags of 732 and 741 exhibited hunched posture on Day 20 and Day 22, respectively; in anti-hPD-1 group (i.e., Donor2-2), mouse with ear tag of 736 died on Day 2, and mice with ear tags of 733 and 735 exhibited hunched posture on Day 20 and Day 24, respectively; in PGc group (i.e., Donor2-3), mice with ear tags of 737, 738, 739, and 740 exhibited hunched posture on Day 20.

Tumor Volume: Tumor volume (TV) was calculated using the following formula:

$$\text{Volume} = (\text{Length} \times \text{Width}^2) / 2.$$

Starting from Day 6, measurements were taken three times per week (Monday, Wednesday, Friday), with the same technician performing all measurements to minimize measurement error.

Animal Body Weight: Mouse body weight was recorded concurrently with tumor volume measurements after grouping.

### 4.5 Sample Collection

Blood Collection: On Day 21 after PBMC inoculation, approximately 100 μL of peripheral blood was collected from each mouse via the retro-orbital bleeding into 1.5 mL EDTA anticoagulant tubes, and then shipped at room temperature by courier to the Institute of Biochemistry, Chinese Academy of Sciences for analysis.

### 4.6 Euthanasia Criteria

During the experiment, mice were euthanized if one or more of the following conditions occurred:

1) Tumor volume in a single animal within a group exceeded 3000 mm$^3$.
2) Tumor exhibited ulceration, necrosis, or infection.
3) Animal showed abnormal mobility or paralysis.
4) Animal body weight decreased by more than 20% compared to its bodyweight at first dosing.

### 5. Statistical Analysis Methods

Efficacy results were presented as mean ± S.E.M. Comparisons between different groups were performed using ANOVA or other appropriate statistical methods. Differences were considered statistically significant when $p < 0.05$.

### 6. Abbreviations

Common abbreviations used in the efficacy study were listed below:

| Abbreviation | Definition |
| --- | --- |
| BID | Twice daily |
| QD | Every day |
| Q2D | Every other day |
| Q3D | Every three days (one day dosing and 2 days off) |
| Q4D | Every four days (one day dosing and 3 days off) |
| BIW | Twice weekly |
| QW | Every week |
| Q3W | Every three weeks |
| i.p. | Intraperitoneal (ly) |
| i.v. | Intravenous(ly) |
| p.o. | Oral(ly) |
| S.C. | Subcutaneous(ly) |
| ANOVA | Analysis of variance |
| TV | Tumor volume |
| BW | Body weight |
| BWL | Body weight loss |

### 7.Experimental Results

**Evaluation of Anti-Tumor Activity of PGc under the HCC827 PBMC Model**

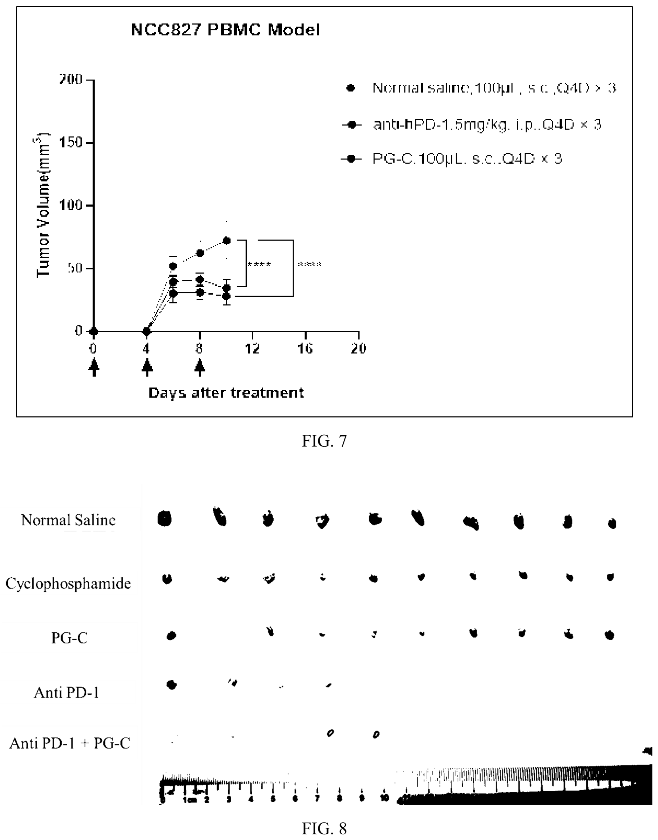

As shown in FIG. 7 and the table below, under the HCC827 PBMC model using 2 donors, both the PGc group and the anti-hPD-1 group significantly inhibited tumor growth compared with the negative control (normal saline) group. In the PGc group, the measured tumor volumes from Day 6 to Day 17 were significantly smaller than those of the negative control group. In the anti-hPD-1 group, the measured tumor volumes from Day 6 to Day 15 were significantly smaller than those of the negative control group. PGc group and anti-hPD-1 group showed similar tumor inhibition, with no significant difference in tumor volume.

| Tumor Volume ($mm^3$) | | Date | 10-18-2022 | 10-22-2022 | 10-24-2022 | 10-26-2022 | 10-28-2022 |
|---|---|---|---|---|---|---|---|
| | | Animal No. | Day 0(T) | Day 4(T) | Day 6(T) | Day 8(T) | Day 10(T) |
| HCC827 , $5\times10^6$, s.c., PBMC Donor:1, $5\times10^6$, *i.p.* | Group 1: Normal saline, 100μL, s.c., Q4D × 3 | 724 | 0.00 | 0.00 | 42.24 | 46.25 | 44.70 |
| | | 725 | 0.00 | 0.00 | 46.76 | 74.58 | 133.67 |
| | | 727 | 0.00 | 0.00 | 71.76 | 88.11 | 89.54 |
| | | Mean | 0.00 | 0.00 | 53.59 | 69.65 | 89.30 |
| | | SD | 0.00 | 0.00 | 15.90 | 21.36 | 44.49 |
| | | SEM | 0.00 | 0.00 | 6.49 | 8.72 | 18.16 |
| | Group 2: anti-hPD-1, 5mg/kg , i.p., Q4D × 3 | 728 | 0.00 | 0.00 | 45.56 | 51.07 | 49.00 |
| | | 729 | 0.00 | 0.00 | 52.58 | 56.56 | 51.41 |
| | | 730 | 0.00 | 0.00 | 38.37 | 31.84 | 18.27 |
| | | Mean | 0.00 | 0.00 | 45.51 | 46.49 | 39.56 |
| | | SD | 0.00 | 0.00 | 7.11 | 12.98 | 18.48 |
| | | SEM | 0.00 | 0.00 | 8.31 | 8.84 | 7.55 |
| | | TGI (%) | / | / | 15.08 | 33.25 | 55.70 |
| | Group 3: PGc, 100μL, s.c., Q4D × 3 | 721 | 0.00 | 0.00 | 35.53 | 34.84 | 14.24 |
| | | 723 | 0.00 | 0.00 | 23.01 | 26.88 | 36.35 |
| | | 726 | 0.00 | 0.00 | 20.10 | 23.49 | 24.31 |
| | | 731 | 0.00 | 0.00 | 22.90 | 24.06 | 25.68 |
| | | Mean | 0.00 | 0.00 | 25.39 | 27.32 | 25.15 |
| | | SD | 0.00 | 0.00 | 6.90 | 5.23 | 9.04 |
| | | SEM | 0.00 | 0.00 | 3.08 | 2.14 | 6.43 |
| | | TGI (%) | / | / | 52.63 | 60.78 | 71.84 |
| HCC827 , $5\times10^6$, s.c., PBMC Donor:2, $5\times10^6$, *i.p.* | Group 4: Normal saline, 100μL, s.c., Q4D × 3 | 732 | 0.00 | 0.00 | 34.40 | 38.42 | 40.48 |
| | | 734 | 0.00 | 0.00 | 39.69 | 41.94 | 43.78 |
| | | 741 | 0.00 | 0.00 | 78.06 | 84.95 | 82.15 |
| | | Mean | 0.00 | 0.00 | 50.72 | 55.10 | 55.47 |
| | | SD | 0.00 | 0.00 | 23.83 | 25.90 | 23.17 |
| | | SEM | 0.00 | 0.00 | 9.73 | 10.58 | 9.46 |
| | Group 5: anti-hPD-1, 5mg/kg , i.p., Q4D × 3 | 733 | 0.00 | 0.00 | 30.05 | 31.82 | 19.14 |
| | | 735 | 0.00 | 0.00 | 30.70 | 35.05 | 34.28 |
| | | 736 | 0.00 | - | - | - | - |
| | | Mean | 0.00 | 0.00 | 30.38 | 33.44 | 26.71 |
| | | SD | 0.00 | 0.00 | 0.46 | 2.29 | 10.71 |
| | | SEM | 0.00 | 0.00 | 4.87 | 5.43 | 5.11 |
| | | TGI (%) | / | / | 43.31 | 51.99 | 70.09 |

(continued)

| Tumor Volume (mm³) | | Date | 10-18-2022 | 10-22-2022 | 10-24-2022 | 10-26-2022 | 10-28-2022 |
|---|---|---|---|---|---|---|---|
| | | Animal No. | Day 0(T) | Day 4(T) | Day 6(T) | Day 8(T) | Day 10(T) |
| | Group 6: PGc, 100μL, s.c., Q4D × 3 | 737 | 0.00 | 0.00 | 36.32 | 32.31 | 31.35 |
| | | 738 | 0.00 | 0.00 | 31.77 | 32.76 | 27.78 |
| | | 739 | 0.00 | 0.00 | 40.82 | 39.53 | 32.82 |
| | | 740 | 0.00 | 0.00 | 33.08 | 35.97 | 32.40 |
| | | Mean | 0.00 | 0.00 | 35.50 | 35.14 | 31.09 |
| | | SD | 0.00 | 0.00 | 4.03 | 3.35 | 2.29 |
| | | SEM | 0.00 | 0.00 | 2.02 | 3.30 | 7.17 |
| | | TGI (%) | / | / | 33.75 | 49.54 | 65.19 |

Conclusion: The PBMC-humanized mice bearing HCC827 xenograft model demonstrated that the PGc biological injection achieved a comprehensive tumor inhibition rate of 68.515%.

**Example 6: Anti-Tumor Efficacy Study of PGc Samples**

**1 Objective**

Evaluation of the anti-tumor efficacy of PGc samples, as well as a preliminary small-scale study on tumor inhibition in combination with anti-mouse PD-1.

**2 Materials and Equipment**

2.1 Test Samples:

2.1.1 Name: PGc (same formulation as described in Example 1)

2.1.2 Supplier: PuGong Biotech (Hangzhou) Co., Ltd.

2.2 Reagents and Materials:

2.2.1 Cell Culture Medium: DMEM (Gibco, Cat. No. C11995500BT, Lot No. 8122010); Serum (YEASEN, Cat. No. 40130ES76, Lot No. 504102121).

2.2.2 Negative and Positive Control Solvent: Sterile Normal Saline (NS), Beyotime, Product No. ST341-500mL.

2.2.3 Positive Control: Cyclophosphamide for Injection (CTX), Baxter International Inc., Lot No. 0G391A.

2.2.4 Combination Therapy: anti-mouse PD-1 (BioXcell, Cat. No. BE0146, Lot No. 695318A1, 599016M2C); Dilution Buffer (BioXcell, Cat. No. IP0070, Lot No. 710920M1).

2.3 Tumor Source:

Mouse Lewis lung carcinoma (LLC) cells, obtained from the Cell Bank of the Chinese Academy of Sciences.

2.4 Experimental Animals

2.4.1 Source: C57BL/6 mice were supplied by Shanghai SLAC Laboratory Animal Co, Ltd. with Certificate No.: 20170005068458; CEMCS Laboratory Animal Use License No. : SYXK (Hu) 2018-0007

2.4.2 Body Weight: Average 19 g

2.4.3 Gender: Female

2.4.4 Number of Animals: Negative control group 10; Positive control group 10; PG-B sample group 10; PGc sample group 10; Anti-PD-1 control group 5; PGc + anti-PD-1combination group 5.

2.5 Instruments and Equipment:

FENYE inverted biological microscope, Model: ZLD200-37T; Heal Force biological safety cabinet, Model: II, A2; Thermo $CO_2$ cell incubator, Model: B

B15

3 Efficacy Study Protocol

| Efficacy Study Protocol | | | | | |
|---|---|---|---|---|---|
| Group | Sample | Dosing Schedule | Dose | Administration Site | Termination |
| Negative Control Group | Normal Saline (NS) | First dosing on day 1 after tumor cell inoculation, sc◊3q3d | 0.1mL/mouse /dose | Subcutaneous injection around and near the tumor site | Tumors were excised and weighed on day 8 after tumor cell inoculation |
| PGc Group | PGc | First dosing on day 1 after tumor cell inoculation, sc◊3q3d | 0.1mL/mouse /dose | Subcutaneous injection around and near the tumor site | |
| ①PGc | ①PGc | ①First dosing on day 1 after tumor cell inoculation, sc03q3d ②First dosing on day 1 after tumor cell inoculation, ip03q3d | ①0.1mL/mouse/dose ②0.6mg/mouse/dose | ①Subcutaneous injection around and near the tumor site ②Mouse Intraperitoneal (i.p.) | |
| ②anti PD-1 Combination Group | ②anti PD-1 | | | | |
| Anti-PD-1 Control Group | Anti PD-1 | First dosing on day 1 after tumor cell inoculation, ip03q3d | 0.6mg/mouse/ dose | Mouse Intraperitoneal (i.p.) | |
| Positive Control Group | Cyclophosphamide (CTX) | First dosing on day 1 after tumor cell inoculation, ip07qd | 30mg/kg/dose | Mouse Intraperitoneal (i.p.) | |

## 4. Main Experimental Procedure

4.1 LLC Cell Culture

The LLC cells were cultured in DMEM medium containing 10% high-quality fetal bovine serum (FBS) and maintained in a cell incubator with 5% $CO_2$ at 37°C.

4.2 Subcutaneous Tumor Formation

The LLC cells (tumor source) in the logarithmic growth phase were collected, and 0.1 mL of cell suspension (approximately $1\times 10^6$ cells) was inoculated subcutaneously into the axillary region of the corresponding mice.

4.3 Randomization

On day 1 after tumor cell inoculation, all mice were randomly grouped before drug administration.

4.4 Animal Dosing

Dosing was performed according to the design of the pharmacological study protocol.

4.5 Termination of the Test

On day 10 after tumor cell inoculation, the experiment was terminated, and tumors in each group of animals were excised and weighed.

**5 Results and Analysis**

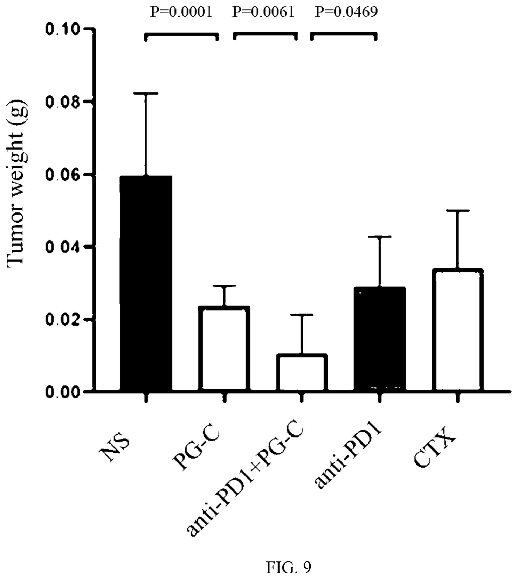

The tumor image was shown in FIG. 8. Statistical Analysis and Tumor Inhibition Rate Calculation was as follows: Data were statistically analyzed using GraphPad Prism software. The tumor inhibition rate was calculated based on changes in tumor size using the following formula: Tumor Inhibition Rate (%) = [(Average tumor weight of control group - Average tumor weight of treatment group) / Average tumor weight of control group] $\times$ 100%. The results were presented in the table below and FIG. 9.

| Statistical analysis and tumor inhibition rate calculation | | | | | |
|---|---|---|---|---|---|
| Group | Sample | Number of Animals (n) Start/End | Tumor Weight (g) $X\pm SD$ | Statistical Analysis | Tumor Inhibition Rate |
| Negative Control Group | Normal saline (NS) | 10/10 | 0.06±0.02 | / | / |
| PGc Group | PGc | 10/10 | 0.02±0.01 | P=0.0001 * | 60.11% |
| ①PGc ②anti PD-1 Combination Group | ①PGc ②Anti PD-1 | 5/5 | 0.01±0.01 | P=0.0005* | 82.27% |
| Anti PD-1 Control Group | Anti PD-1 | 5/5 | 0.03±0.01 | P=0.0159* | 51.58% |
| Positive Control Group | Cyclophosphamide (CTX) | 10/10 | 0.03±0.02 | P=0.0091 * | 42.80% |
| Note: *Comparison between the treatment group and the negative control group. | | | | | |

The results showed that under the Lewis lung carcinoma (LLC) tumor model in C57BL/6 mice, when the PGc biological injection was used alone, the tumor inhibition rate reached 60.11%, while when the PGc biological injection was used in combination with PD-1, the tumor inhibition rate reached 82.27%.

**Example 7: Flow Cytometry Analysis of Activation of Antitumor Immune Cells induced by PGc Samples**

**1 Objective** of **the Experiment**

To analyze the activation of antitumor immune cells induced by the PGc samples using flow cytometry, thereby providing insights into the underlying antitumor mechanism of the PGc samples.

**2 Experimental Materials and Equipment**

2.1 Test Samples:

2.1.1 Name: PGc (same formulation as described in Example 1)

2.1.2 Supplier: PuGong Biotech (Hangzhou) Co., Ltd.

2.2 Reagents and Materials:

2.2.1 Cell Culture Medium: DMEM (Gibco, Cat. No. C11995500BT, Lot No. 8122010); Serum (Gemini, Cat. No. 900-123, Lot No. A24F02G).

2.2.2 Negative and Positive Control Solvent: Sterile Normal Saline (NS), Beyotime, Product No. ST341-500mL.

2.2.3 Positive Control: Cyclophosphamide for Injection (CTX), Baxter International Inc., Lot No. 0G391A.

2.2.4 Tumor Tissue Lymphocyte Isolation Reagents: Collagenase IV (Sigma, Cat. No. C-5138); DNase I (Roche, Cat. No. 143582); Percoll (GE Healthcare, Cat. No. 17-0891-01).

2.2.5 Antibodies for Flow Cytometry: anti-CD3$\varepsilon$ FITC, eBioscience, Cat. No. 11-0031-82; anti-CD4 PerCP-Cy5.5, eBioscience, Cat. No. 45-0042-82; anti-CD8a APC, eBioscience, Cat. No. 17-0081-83; anti-B220 FITC, eBioscience, Cat. No. 11-0452-82; anti-CD45 PerCP-Cy5.5, BD, Cat. No. 561047; anti-NK1.1 PE, eBioscience, Cat. No. 12-5941-81.

2.3 Tumor Source:

Mouse Lewis lung carcinoma (LLC) cells, obtained from the Cell Bank of the Chinese Academy of Sciences.

2.4 Experimental Animals

2.4.1 Source: C57BL/6 mice were supplied by Shanghai SLAC Laboratory Animal Co, Ltd. with Certificate No.: 20170005064721; CEMCS Laboratory Animal Use License No.: SYXK (Hu) 2018-0007.

2.4.2 Age: 8 weeks old.

2.4.3 Gender: Female.

2.4.4 Number of Animals: PGc sample group (1 group), negative control group (1 group), and positive control group (1 group), with 10 mice per group.

2.5 Instruments and Equipment:

Flow Cytometer: CytoFLEX, Beckman.

**3 Flow Cytometry Analysis Protocol**

Drug administration was discontinued on Day 10 after tumor cell inoculation. Tumors were excised from both the NS control group and the PGc-treated group for flow cytometry analysis of immune cells.

| Flow Cytometry Analysis Protocol | | | | | |
|---|---|---|---|---|---|
| Group | Sample | Dosing Schedule | Dose | Administrat ion Site | Termination |
| Negative Control Group | Normal Saline (NS) | First dosing on day 1 after tumor cell inoculation, sc◊3q3d | 0.1mL/mouse/dos e | Subcutaneo us injection around and near the tumor site | On day 10 after tumor cell inoculation, tumors were excised, and flow cytometry analysis of immune cells was performed for both the NS control group and the PGc-treated group. |
| PGc Group | PGc | First dosing on day 1 after tumor cell inoculation, sc◊3q3d | 0.1mL/mouse/dos e | Subcutaneo us injection around and near the tumor site | |
| Positive Control Group | Cyclophos phamide (CTX) | First dosing on day 1 after tumor cell inoculation, ip◊7qd | 30mg/kg/dose | Intraperiton eal injection | |

**4. Main Experimental Procedures:**

4.1 LLC Cell Culture:

The LLC cells were cultured in DMEM medium containing 10% high-quality fetal bovine serum and maintained in a cell incubator with 5% $CO_2$ at 37°C.

4.2 Subcutaneous Tumor Formation

The LLC cells (tumor source) in the logarithmic growth phase were collected, and 0.1 mL of cell suspension (approximately $1 \times 10^6$ cells) was inoculated subcutaneously into the axillary region of the corresponding mice.

4.3 Random Grouping

On day 1 after tumor cell inoculation, all mice were randomly grouped before drug administration.

4.4 Animal Dosing and Termination

Drug administration was carried out according to the experimental design. On the day 10 after tumor cell inoculation, dosing was discontinued, and tumors from the NS control group and the PGc-treated group were excised for analysis.

4.5 Flow Cytometry Analysis

**4.5.1** Isolation of Tumor-Infiltrating Lymphocytes (TILs): Mouse tumors were carefully excised along the tumor margins and placed in a 6 cm dish containing PBS. A small portion of the tumors was collected and transferred into a tube, and approximately 5-6 times the tumor volume of 1 mg/mL collagenase IV and 1 mg/mL DNase I was added. The tube was sealed with parafilm and incubated on a shaker for digestion at 37°C for 0.5-1 hour. A 70 μm filter membrane was placed on a 6 cm dish, and the tumor digestion mixture was passed through the filter membrane. The tissues were gently ground with a pestle to allow a single-cell suspension to flow into the dish. The single-cell suspension was then transferred into a 15 mL centrifuge tube and centrifuged at $1200 \times$ g for 5 minutes. The supernatant was discarded, and the pellet was resuspended in 3 mL of 35% Percoll. In a new 15 mL centrifuge tube, 3 mL of 70% Percoll was added, and the previous 3 mL of cell suspension was carefully layered on top to avoid mixing. The tube was centrifuged at 2500 rpm for 20 minutes. The middle lymphocyte layer was collected, washed once with 15 mL PBS, centrifuged at 2000 rpm for 5 minutes, and resuspended in PBS.

4.5.2 Flow Cytometry Staining: The resuspended cells were added to centrifuge tubes containing flow cytometry antibodies (1 μL antibody per sample), mixed gently by pipetting to ensure even distribution, with a total volume of 50 μL. Samples were incubated at 4°C for 45-60 minutes in the dark. After incubation, 1 mL of PBS was added to each centrifuge tube and mixed thoroughly by pipetting, followed by centrifugation in a horizontal centrifuge at 1000 rpm for 5 minutes. The supernatant was discarded, and the cell pellet was resuspended in 200 μL PBS. The samples were transferred to flow cytometry tubes, protected from light, and analyzed by flow cytometry.

**5 Experimental Results and Analysis**

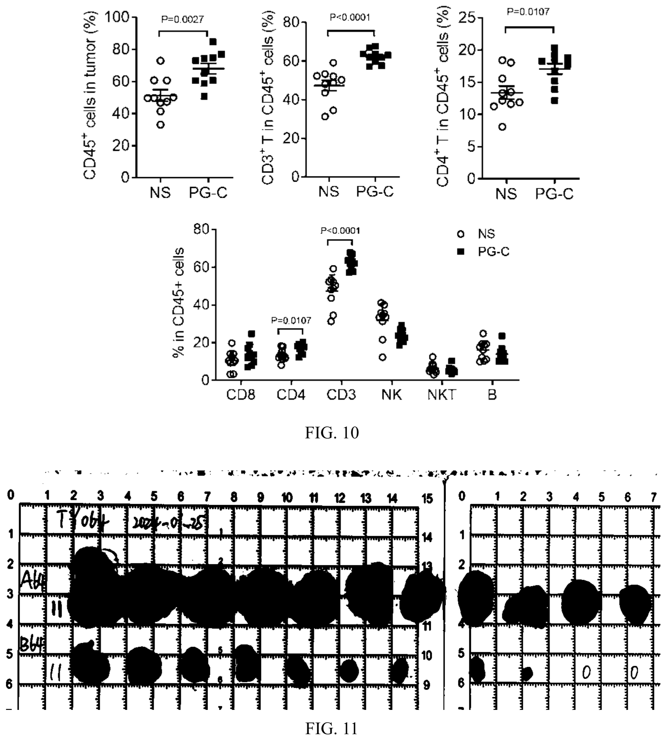

Flow cytometry analysis of tumor-infiltrating cells from the NS and PGc groups revealed statistically significant differences in CD45+ immune cells, CD3+ T cells, and CD4+ T cells, with p-values of $P = 0.0027$, $P < 0.0001$, and $P = 0.0107$, respectively. No statistically significant differences were observed in CD8+ T cells, NK cells, NKT cells, or B cells (see FIG. 10).

**Example 8: Verification of the Anti-Tumor Efficacy of PGc-36 Against B16f0 Melanoma**

Tumor Source: B16F0 melanoma cells were purchased from Zhejiang Meisen Biotechnology Co., Ltd. Tumor models were established by subcutaneous implantation in B/C mice. Tumor culture and tumor-bearing mice were prepared by Hangzhou Medical College.

Animal information: Age: 8 weeks old; Gender: Female.
Number of Animals: PGc sample group (1 group) and negative control group (1 group), with 11 mice per group.

The dosing regimen and dose setting were shown in the table below:

In the table, "A64" referred to the negative control group (sodium chloride injection), and "B64" referred to PGc-1208, whose formulation was identical to that in Example 1. The different batch numbers represented different production batches.

The unit of all injection doses was milliliters (mL). "PGc 0.1 + 0.1" indicated that 0.1 mL of PGc was injected at two separate sites, and the same applied to other examples.

| Preliminary Animal Efficacy Study Protocol for Repeat Validation against B16F0 Subcutaneous Melanoma Tumor -- 3C57 (TY064), January 8 | | | | | | | | | | | | |
|---|---|---|---|---|---|---|---|---|---|---|---|---|
| Cell source: PuGong | | Inoculation: Medical College | | Tumor implantation | Dosin g 1 | Dosin g 2 | Dosin g3 | Dosin g 4 | Dosin g 5 | Dosin g 6 | Dosin g 7 | Dosi ng 8 | Disse ction |
| Experiment Schedule | | | | 0 | 1 | 3 | 5 | 7 | 9 | 11 | 13 | 15 | 17 |
| Gro up | Contr ol | Qua ntity | Administrati on Route | Jan 8 | Jan 9 | Jan 11 | Jan 13 | Jan 15 | Jan 17 | Jan 19 | Jan 21 | Jan 23 | Jan 25 |
| A6 4 | NS | 11 | Subcutaneo us | B16F0 | NS 0.2 | NS 0.2 | NS 0.2 | NS 0.2 | NS 0.2 | NS 0.2 | NS 0.2 | | Disse ction |
| B6 4 | PG-c-12-08 | 11 | Subcutaneo us | B16F0 | PGc0. 1+0.1 | PGc0. 1+0.1 | PGcO. 1+0.1 | PGcO. 1+0.1 | PGcO. 1+0.1 | PGc0. 1+0.1 | PGc0. 1+0.1 | | Disse ction |

The tumor image was shown in FIG. 11. The tumor weight data (unit: g) and tumor inhibition rate were shown in the table below. "Total" represented the total tumor weight of all eleven mice.

|  | 1 | 2 | 3 | 4 | 5 | 6 | 7 | 8 | 9 | 10 | 11 | Total (g) | TIR |
|---|---|---|---|---|---|---|---|---|---|---|---|---|---|
| A64 | 2.57 | 2.3 | 2.29 | 2.06 | 1.64 | 1.64 | 1.3 | 1 | 0.93 | 0.88 | 0.46 | 17.07 | |
| B64 | 0.6 | 0.4 | 0.4 | 0.37 | 0.15 | 0.1 | 0.1 | 0.08 | 0.01 | 0 | 0 | 2.21 | 0.8705 |

The results showed that the PGc biological injection could effectively inhibit the growth of melanoma.

**Example 9: Verification of the Anti-Tumor Efficacy of PGc-F31\B36 against RM-1 Prostate Cancer**

Tumor Source: The RM-1 murine prostate cancer cell line was used in this experiment. Tumors were subcutaneously implanted in C57 mice, and tumor culture as well as mouse tumor implantation were conducted by Hangzhou Medical College.
Animal information: Age: 8 weeks; Gender: Male.
Number of Animals: PGc sample group (2 groups), negative control group (1 group), with 5-6 mice per group.
Negative Control Group: Sodium chloride injection. The formula of PGe-B36 was consistent with that in Example 1.

Dosing Regimen and Dose Setting:

| Preliminary Animal Efficacy Study Protocol regarding Subcutaneous RM-1 Prostate Cancer -- 3C57 (TY052), December 5 | | | | | | | | | | |
|---|---|---|---|---|---|---|---|---|---|---|
| Cell source: Medical College | | Inoculation: Medical College | | | Tumor implantatio n | Dosing 1 | Dosing 2 | Dosing 3 | Dosing 4 | Dosing 5 | Dissectio n |
| Experiment Schedule | | | | 0 | 1 | 3 | 5 | 7 | 9 | 11 |
| Grou p | Contro 1 | Quantit y | Administratio n Route | Dec 05 | Dec 06 | Dec 08 | Dec 10 | Dec 12 | Dec 14 | Dec 16 |
| A52 | NS | 6 | Subcutaneous | RM-1 | NS 0.1 | NS 0.1 | NS 0.1 | NS 0.1 | NS 0.1 | Tumor weighing |
| C52 | PGc-B36 | 5 | Subcutaneous | RM-1 | PGc0.1+0. 1 | PGcO. 1 | PGc0.1+0. 1 | PGcO. 1 | PGc0.1+0. 1 | Tumor weighing |

The unit of injection dose in the table was milliliters (mL).

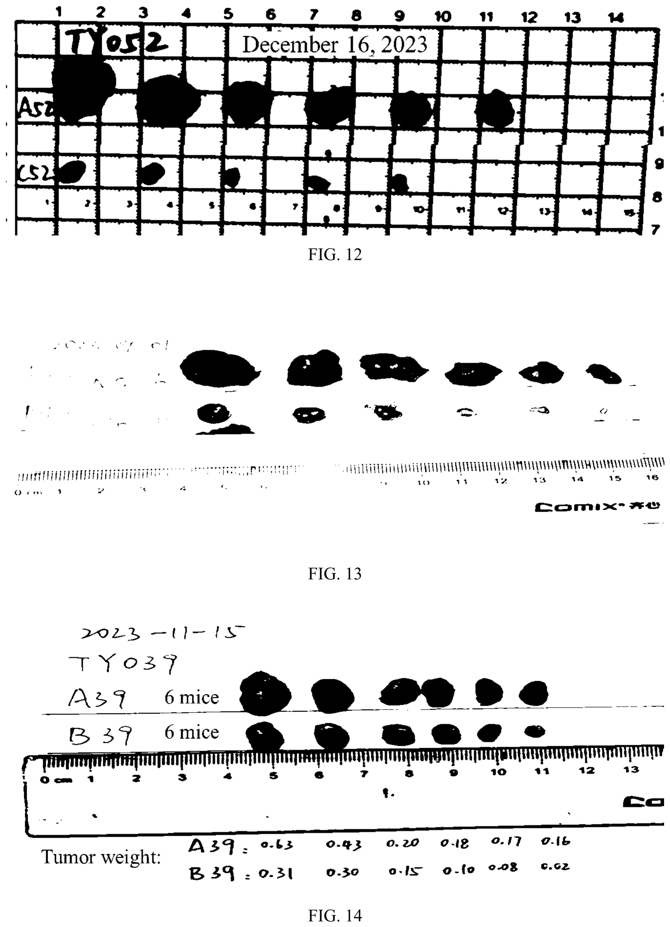

The tumor image was shown in FIG. 12. The tumor weight data (unit: g) and tumor inhibition rate were presented in the table below. The first row (1-6) represented an individual mouse, and the "Total" referred to the total tumor weight of the six mice.

|  | 1 | 2 | 3 | 4 | 5 | 6 | Total | TIR |
|---|---|---|---|---|---|---|---|---|
| A52 | 1.45 | 1.05 | 0.61 | 0.45 | 0.38 | 0.3 | 4.24 |  |
| C52 | 0.09 | 0.08 | 0.03 | 0.03 | 0.02 | 0.044 | 0.294 | 0.93 |

The results indicated that the PGc biological injection could effectively inhibit the growth of prostate tumors.

**Example 10: Verification of the Anti-Tumor Efficacy of PGc against HT1080 Human Sarcoma**

Tumor Source: HT1080 Human sarcoma, subcutaneously implanted in Nude B/C mice. Tumor culture and mouse tumor implantation were conducted by Beijing Charles River Laboratory Animal Technology Co., Ltd.
Animal information: Age: 8 weeks; Gender: Male.
Negative Control Group: Sodium chloride injection. The formulation of PGc-C16 was consistent with that in Example 1. Different batch numbers represented different production batches.

Dosing Regimen and Dose Setting:

EP 4 706 672 A1

| Preliminary Animal Efficacy Study Protocol regarding fibrosarcoma in Nude Mice -- B/C (TY023), August 18 | | | | | | | | | | | | | | | | | |
|---|---|---|---|---|---|---|---|---|---|---|---|---|---|---|---|---|---|
| | | | | Tumor implant ation | | Dosi ng 1 | Dosi ng 2 | Dosi ng 3 | Dosi ng 4 | Dosi ng 5 | Dosi ng 6 | Dosi ng 7 | Dosi ng 8 | Dosi ng 9 | Disse ction |
| Experiment Schedule | | | | 0 | 1 | 2 | 4 | 6 | 8 | 10 | 12 | 14 | 16 | 18 | 20 |
| Gro up | Con trol | Quan tity | Administ ration Route | Aug 16 | Aug 17 | Aug 18 | Aug 20 | Aug 22 | Aug 24 | Aug 26 | Aug 28 | Aug 30 | Sep 1 | Sep 3 | Sep 5 |
| A2 3 | NS | 6 | Subcutan eous | HT108 0 inocula tion | Ship ment | NS 0.05 | NS 0.05 | NS 0.05 | NS 0.05 | NS 0.05 | NS 0.05 | NS 0.05 | NS 0.05 | NS 0.05 | Tumo r weigh ing |
| B2 3 | PGc C16 | 6 | Subcutan eous | Sarcom a inocula tion | | PGc 0.05 | PGc 0.05 | PGc 0.05 | PGc 0.05 | PGc 0.05 | PGc 0.05 | PGc 0.05 | PGc 0.05 | PGc 0.05 | PGc0. 05 |

38

**EP 4 706 672 A1**

The tumor image was shown in FIG. 13. The tumor weight data (unit: g) and tumor inhibition rate were shown in the table below. Numbers 1-6 in the first row of the table represented different mice, respectively, and "Total" referred to the total tumor weight of all six mice.

|             | 1    | 2    | 3    | 4    | 5    | 6    | Total | TRI    |
|-------------|------|------|------|------|------|------|-------|--------|
| A23 Control | 2.08 | 1.42 | 0.94 | 0.77 | 0.55 | 0.21 | 5.97  |        |
| B23         | 0.36 | 0.24 | 0.23 | 0.05 | 0.03 | 0    | 0.91  | 0.8476 |

The results showed that the PGc biological injection could effectively inhibit the growth of sarcomas.

**Example 11: Verification of the Anti-Tumor Efficacy of PGc against 4T1 Breast Cancer**

Tumor Source: The 4T1 murine breast cancer cell line was used in this experiment. Tumors were subcutaneously implanted in C57 mice, and tumor culture as well as mouse tumor implantation were conducted by Hangzhou Medical College.
Animal information: Age: 8 weeks; Gender: Male.
Negative Control Group: Sodium chloride injection. The formulation of PGc-B32N was consistent with that in Example 1. Different batch numbers represented different production batches.

Dosing Regimen and Doe Setting:

| Preliminary Animal Efficacy Study Protocol regarding Murine 4T1 Breast Cancer -- B/C (TY039), November 1 | | | | | | | | | | | | |
|---|---|---|---|---|---|---|---|---|---|---|---|---|
| Cell source: Medical College | | Inoculation: Medical College | | Tumor implantation | Dosin g 1 | Dosin g 2 | Dosin g3 | Dosin g 4 | Dosin g 5 | Dosin g 6 | Dosin g 7 | Dissection |
| Experiment Schedule | | | | 0 | 1 | 3 | 5 | 7 | 9 | 11 | 13 | 15 |
| Group | Control | Quan tity | Administratio n Route | Oct 31 | Nov 1 | Nov 3 | Nov 5 | Nov 7 | Nov 9 | Nov 11 | Nov 13 | Nov 15 |
| A39 | NS | 6 | Subcutaneous | 4T1 | NS 0.1 | NS 0.1 | NS 0.1 | NS 0.1 | NS 0.1 | NS 0.1 | NS0. 1 | Tumor weighing |
| B39 | PGc-B32N | 6 | Subcutaneous | breast cancer | PGcO .1 | PGc0 .1 | PGc0 .1 | PGc0 .1 | PGc0 .1 | PGcO .1 | PGcO .1 | Tumor weighing |

The tumor image was shown in FIG. 14. The tumor weight data (unit: g) and tumor inhibition rate were shown in the table below. Numbers 1-6 in the first row of the table represented different mice, respectively, and "Total" refers to the total tumor weight of all six mice.

|  | 1 | 2 | 3 | 4 | 5 | 6 | Total | TIR |
|---|---|---|---|---|---|---|---|---|
| A39 | 0.63 | 0.43 | 0.2 | 0.18 | 0.17 | 0.16 | 1.77 |  |
| B39 | 0.31 | 0.3 | 0.15 | 0.1 | 0.08 | 0.02 | 0.96 | 0.4576 |

The results indicated that the PGc biological injection could effectively inhibit the growth of breast cancer.

**Example 12: Verification of the Anti-Tumor Efficacy of PGc-C16 against Pan02 Pancreatic Cancer**

Tumor source: The Pan 02 cell line, subcutaneously implanted in B/C mice. Tumor culture and mouse tumor implantation were conducted by Beijing Charles River Laboratory Animal Technology Co., Ltd.
Animal information: Age: 8 weeks; Gender: Male.
Negative Control Group: Sodium chloride injection. The formulation of PGc-C16 was consistent with that in Example 1. Different batch numbers represented different production batches.

Dosing Regimen and Dose Setting:

| | | | | Tumor implantation | | Dosing 1 | Dosing 2 | Dosing3 | Dosing 4 | Dosing 5 | Dosing 6 | Dosing 7 | Dosing 8 | Dosing 9 | Dosing 10 | Dosing 11 | Dosing 12 | Dissection |
|---|---|---|---|---|---|---|---|---|---|---|---|---|---|---|---|---|---|---|
| | Preliminary Animal Efficacy Study Protocol regarding Pan02 Pancreatic Cancer -- B/C (TY029), September 1 | | | | | | | | | | | | | | | | | |
| Experiment Schedule | | | | 0 | 1 | 2 | 4 | 6 | 8 | 10 | 12 | 14 | 16 | 18 | 20 | 22 | 24 | 26 |
| Group | Control | Quantity | Administration Route | Aug 30 | Aug 31 | Sep 1 | Sep 3 | Sep 5 | Sep 7 | Sep 9 | Sep 11 | Sep 13 | Sep 15 | Sep 17 | Sep 19 | Sep 21 | Sep 23 | Sep 25 |
| A 29 | NS | 5 | Subcutaneous | Pan02 | Shipment | NS 0.1 | NS 0.1 | NS 0.1 | NS 0.1 | NS 0.1 | NS 0.1 | NS 0.1 | NS 0.1 | NS 0.1 | NS 0.1 | NS 0.1 | NS 0.1 | Tumor weighing |
| B 29 | PGc-C16 | 5 | Subcutaneous | pancreatic cancer | | PGc0.1 | PGc0.1 | PGc0.1 | PGc0.1 | PGc0.1 | PGc0.1 | PGc0.1 | PGc0.1 | PGc0.1 | PGc0.1 | PGc0.1 | PGc0.1 | Tumor weighing |

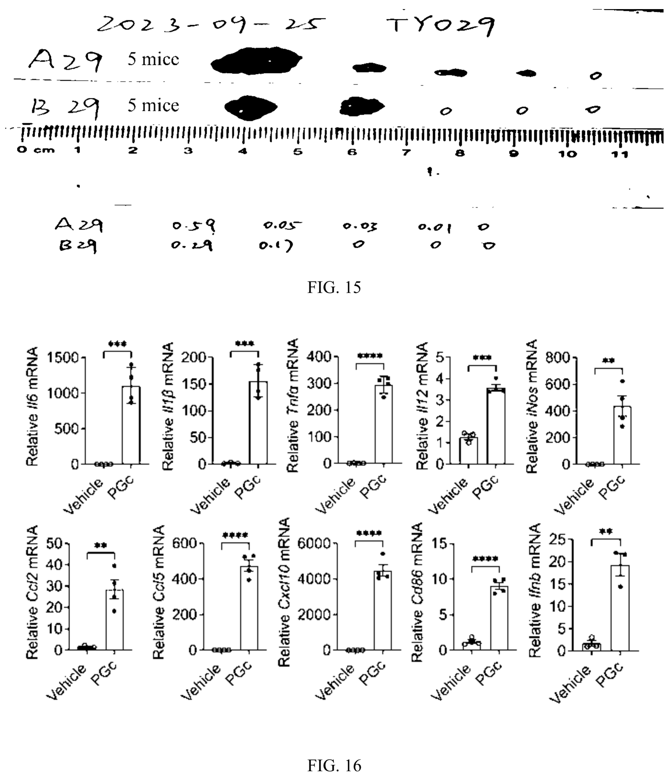

The tumor image was shown in FIG. 15. The tumor weight data (unit: g) and tumor inhibition rate were shown in the table below. Numbers 1-6 in the first row of the table represented different mice, respectively, and "Total" referred to the total tumor weight of all six mice.

|      | 1    | 2    | 3    | 4    | 5 | Total | TIR    |
|------|------|------|------|------|---|-------|--------|
| A29  | 0.59 | 0.05 | 0.03 | 0.01 | 0 | 0.68  |        |
| B29  | 0.29 | 0.17 | 0    | 0    | 0 | 0.46  | 0.3235 |

The results indicated that the PGc biological injection could effectively inhibit the growth of pancreatic cancer.

**Example 13: Evaluation of the Potential Toxicity of the PGc Broad-Spectrum Antitumor Preparation**

The objective of this study was to evaluate the potential toxic responses in cynomolgus monkeys following repeated subcutaneous administration of the PGc broad-spectrum antitumor injection over three months (administered once every 3 days in the first week, with a total of 3 doses; then once a week for the subsequent period, with a total of 12 doses) and to identify possible target organs of toxicity. The formulation of the PGc broad-spectrum antitumor injection was identical to that described in Example 1.

Two male cynomolgus monkeys received subcutaneous injections of high and low doses (4.0 mL/animal or 2.0 mL/animal) of the PGc broad-spectrum antitumor injection, respectively, on Days 1, 4, 7, 14, 21, 28, 35, 42, 49, 56, 63, 70, 77, 84, and 91. At the end of the administration period (Day 93), the animals were subjected to necropsy. During the study, the following parameters were evaluated: clinical observation, body weight, food consumption, body temperature (rectal temperature), electrocardiogram (ECG), body surface temperature, hematology, blood coagulation, plasma biochemistry, immunoglobulins and complement, lymphocyte immunophenotyping, urinalysis, cytokine, and gross pathological observation. The evaluation methods for all parameters were standard and well-established in the field; therefore, they were not described in detail herein.

During the study period, the principal changes observed following administration of the test article (PGc) were observed in clinical observation, body weight, body surface temperature, clinical pathology (including hematology, blood coagulation, plasma biochemistry, immunoglobulins, and lymphocyte immunophenotyping), cytokine, and gross necropsy findings. The main observed changes were summarized in the following table and described below:

Clinical Observation: Following repeated administration, both the high-dose and low-dose groups exhibited multiple palpable nodules (firm on palpation) at the injection sites, and the number of protrusions in the high-dose group was significantly greater than that in the low-dose group. In addition, both animals showed transient erythema at the injection sites after the first administration.

Body Weight: In the high-dose group, slight decreases in body weight were observed on multiple occasions during the mid-to-late stages of administration. Specifically, compared with the pre-dosing baseline (Day -1), animals in the PGc 4.0 mL/animal group showed slight decreases in body weight on multiple occasions during the mid-to-late dosing period (Days 21, 49, 56, 63, 70, 77, and 91), with a maximum reduction of approximately 7%. Throughout the study, animals in the PGc 2.0 mL/animal group exhibited no significant body weight changes.

Body Surface Temperature: In the high-dose group, a mild increase in body surface temperature was observed on the days of the 3rd and 5th administrations (8 and 16 hours after administration, respectively).

Hematology: In both dose groups, elevated levels of WBC (white blood cells) and NEUT (neutrophils) were observed on the second day following the 3rd, 6th, 10th, and 15th administrations; however, no clear dose-dependent relationship was noted.

Blood Coagulation: In both dose groups, elevated levels of FIB (Days 8, 29, 57, and 92), APTT (Days 8, 57, and 92), and PT (Day 8) were observed on the second day following the 3rd, 6th, 10th, and 15th administrations. In the PGc 2.0 mL/animal group, increases in FIB (Days 8, 29, 57, and 92) and APTT (Day 57) were also observed at the same time points.

Plasma Biochemistry and Immunoglobulins: In both dose groups, on the second day following the 3rd, 6th, 10th, and 15th administrations (Days 8, 29, 57, and 92), decreased ALB (albumin) levels and increased GLOB (globulin) levels (resulting in a reduced A/G ratio) were observed. In addition, animals in the high-dose group showed slight increases in IgM and/or IgG at the same time points.

Lymphocyte Immunophenotyping: In both dose groups, on the second day following the 3rd, 6th, 10th, and 15th administrations, decreases in CD3$^+$CD8$^+$ cells and increases in the CD4$^+$/CD8$^+$ ratio were observed, along with a slight increase in CD3$^-$CD16$^+$ cells (noted only after the 3rd administration). Furthermore, in the high-dose group, a marked increase in CD3$^-$CD11b$^+$ cells was observed after the 15th administration.

Cytokines: In both dose groups, elevations in IL-6 were observed at 2 and 24 hours after administration on the days of the 1st, 6th, 10th, and 15th administrations (Days 7, 28, 56, and 91).

Gross Necropsy Findings: During necropsy, animals in both dose groups exhibited only multifocal nodules at the sites of the last administration (cervical-dorsal region).

No test article-related changes were observed in other evaluated parameters, including food consumption, rectal temperature, electrocardiogram (ECG), and complement.

| Dose | Start Date | Blood Coagulation | | Plasma Biochemistry | | | Special Immune Indicators | | Hematology | | Lymphocyte Immunophenotype | | | |
|---|---|---|---|---|---|---|---|---|---|---|---|---|---|---|
| | | Activated Partial Thrombo plastin Time | Fibrin ogen | Alb umin | Glo bulin | Albu m/Globu l | Immun oglobul in G | Immuno globulin M | White Blood Cell Count | Neutr ophils | | | | |
| | | APTT (Seconds) | FIB (g/L) | ALB (g/L) | GLOB (g/L) | A/G | IgG (g/L) | IgM (g/L) | WBC (10$^9$/L) | NEUT (10$^9$/L) | CD3+CD8+ | CD4+/CD8+ | CD3-CD16+ | CD3-CD11b+(%) |
| 2.0 ml/Animal | 12 | 16.9 | 2.26 | 48.0 | 28.0 | 1.7 | / | / | 12.07 | 6.97 | / | / | / | / |
| | -3 | 17.5 | 2.18 | 46.9 | 26.7 | 1.8 | 8.29 | 0.08 | 10.00 | 3.63 | 17.99 | 1.77 | 5.08 | 9.14 |
| | 8 | 21.2 | 4.96 | 40.0 | 38.2 | 1.0 | 7.47 | 1.19 | 20.57 | 17.19 | 9.30 | 2.29 | 11.41 | 16.8 |
| | 29 | 19.7 | 3.93 | 38.4 | 34.2 | 1.1 | 8.80 | 1.00 | 26.40 | 21.50 | 9.89 | 2.42 | 8.69 | 13.84 |
| | 57 | 23.6 | 3.84 | 40.2 | 35.7 | 1.1 | 9.07 | 0.94 | 23.88 | 20.24 | 8.90 | 3.04 | 8.23 | 13.84 |
| | 92 | 20 | 3.60 | 36.9 | 37.2 | 1.0 | 8.23 | 0.95 | 19.13 | 14.93 | 8.54 | 2.77 | 8.30 | 12.81 |
| 4.0 ml/Mice | 12 | 24.1 | 3.03 | 46.3 | 34.3 | 1.3 | / | / | 9.92 | 5.60 | / | / | / | / |
| | -3 | 21.1 | 2.89 | 43.3 | 32.3 | 1.3 | 9.34 | 0.91 | 13.99 | 7.41 | 10.72 | 2.76 | 2.99 | 13.23 |
| | 8 | 38.7 | 4.40 | 35.1 | 48.0 | 0.7 | 9.57 | 1.35 | 22.47 | 18.04 | 7.06 | 3.58 | 7.10 | 14.76 |
| | 29 | 24.7 | 4.58 | 35.9 | 45.7 | 0.8 | 12.72 | 1.13 | 24.58 | 19.13 | 8.50 | 3.42 | 3.40 | 10.98 |
| | 57 | 28.5 | 3.84 | 36 | 41.2 | 0.9 | 11.19 | 1.12 | 22.05 | 18.43 | 4.54 | 5.30 | 3.61 | 10.66 |
| | 92 | 26.4 | 3.96 | 36.7 | 43.4 | 0.8 | 11.51 | 1.29 | 25.23 | 20.45 | 4.87 | 5.71 | 6.74 | 27.46 |

In conclusion, under the conditions of this study, repeated subcutaneous administration of the PGc broad-spectrum antitumor injection at 2.0 and 4.0 mL/animal for 3 months was well tolerated by cynomolgus monkeys in both dose groups. The primary changes observed were local irritation at the injection sites (manifested mainly as nodules) and mild immune activation (reflected by increases in IgM, IgG, WBC count, NEUT count, and IL-6). These results indicated that the PGc broad-spectrum antitumor injection of the present disclosure possessed a favorable safety profile.

**Example 14 Mechanism of Action of the Broad-Spectrum Antitumor Preparation PGc**

1. Experimental Method for PGc-Induced Macrophage Activation

**1) Induction, Isolation, and Culture of Primary Mouse Peritoneal Macrophages:**

Mice aged 8 weeks or older were intraperitoneally injected with 3% thioglycolate aqueous solution at a dose of 3 mL per mouse. Four days later, the mice were euthanized by cervical dislocation or carbon dioxide asphyxiation and immersed in 70% ethanol for 2 minutes for sterilization. Each mouse was placed in a supine position, and a small incision was carefully made on the outer abdomen while keeping the peritoneum intact. Using a 5 mL syringe, 5 mL of DMEM culture medium was injected into the abdominal cavity. The syringe was withdrawn, and the abdomen was gently massaged for approximately 3 minutes. The syringe needle was then reinserted into the abdominal cavity to aspirate as much fluid as possible, which was then transferred into a centrifuge tube. The collected cells were centrifuged at 1000 rpm for 5 minutes, and the supernatant was discarded. The cell pellet was resuspended in an appropriate volume of complete DMEM medium, and the cells were counted. Cells were seeded into 12-well plates at a density of $1 \times 10^6$ cells per well. After overnight incubation, the adherent cells were identified as peritoneal macrophages and used for subsequent experiments.

**2) Stimulation of Macrophages with PGc:**

PGc (with a formulation same as that in Example 1) was diluted 1:100 in complete DMEM culture medium. The medium after the previous overnight macrophage culture was removed and replaced with fresh medium containing PGc. The cells were then incubated for 6 hours.

**3) RNA Extraction, Reverse Transcription, and Quantitative Real-Time RT-qPCR**

RNA Extraction: After cell treatment, the culture medium was discarded, and the cells were washed once with PBS. Then, 1 mL of TRIzol reagent was added to lyse the cells, followed by incubation at room temperature for 5 minutes. Subsequently, 200 $\mu$L of chloroform was added, and the mixture was vigorously vortexed and incubated at room temperature for 3 minutes. The samples were centrifuged at 12,000 rpm for 15 minutes at 4°C. The upper aqueous phase (liquid, approximately 500 $\mu$L) was carefully collected and mixed with an equal volume of isopropanol. After incubation at room temperature for 10 minutes, the mixture was centrifuged again at 12,000 rpm for 10 minutes at 4°C. The RNA pellet was washed once with 1 mL of 75% ethanol, followed by centrifugation at 12,000 rpm for 5 minutes at 4°C. The supernatant was discarded, and the residual liquid was carefully removed using a fine pipette tip. The RNA pellet was air-dried for 5-10 minutes and dissolved in 20 $\mu$L of DEPC-treated double-distilled water (ddH$_2$O). RNA concentration was measured using a NanoDrop spectrophotometer. The extracted RNA was stored at -80°C.

Reverse Transcription: A mixture containing 1$\mu$g of total RNA and 100 ng of Oligo (dN$_6$) was prepared and adjusted to a final volume of 14 $\mu$L with DEPC-treated water. The mixture was incubated at 70°C for 10 minutes, immediately cooled on ice for 2 minutes, and then supplemented with 4 $\mu$L of 5$\times$ reverse transcription buffer, 1$\mu$L of M-MLV reverse transcriptase, and 1$\mu$L of 10 mM dNTPs. The total reaction volume was brought to 20 $\mu$L with DEPC-treated water. The reaction system was incubated at 37°C for 1hour, followed by enzyme inactivation at 72°C for 10 minutes. The resulting reverse transcription product was diluted with 180 $\mu$L of deionized water and then used as cDNA template for qPCR analysis.

Quantitative Real-Time RT-qPCR: The RT-qPCR reaction system had a total volume of 20 $\mu$L, consisting of 3.2 $\mu$L of deionized water, 10 $\mu$L of SYBR Green Master Mix, 6 $\mu$L of diluted cDNA template, and 0.8 $\mu$L each of forward and reverse primers (10 $\mu$M). The thermal cycling conditions were as follows: 95°C for 3 minutes; followed by 40 cycles of 95°C for 10 seconds, 60°C for 30 seconds, and 72°C for 30 seconds, with fluorescence signal acquisition at the end of each cycle. A subsequent melting curve analysis was performed with the following program: 95°C for 1minute, 55°C for 1 minute, and a temperature increase from 55°C to 98°C at 5°C increments every 5 seconds, with fluorescence signal continuously recorded.

The results were shown in FIGs. 16-17. Compared with the control group (Vehicle group), the mRNA expression levels were significantly increased in the PGc-treated group. These results indicated that PGc promoted the activation of M1-type macrophages.

2. Construction of Animal Models

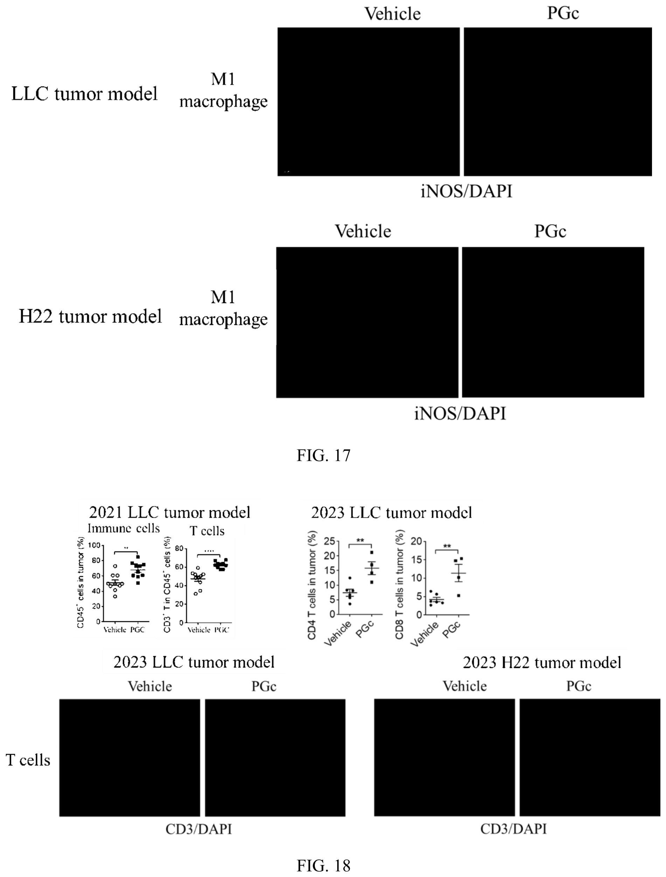

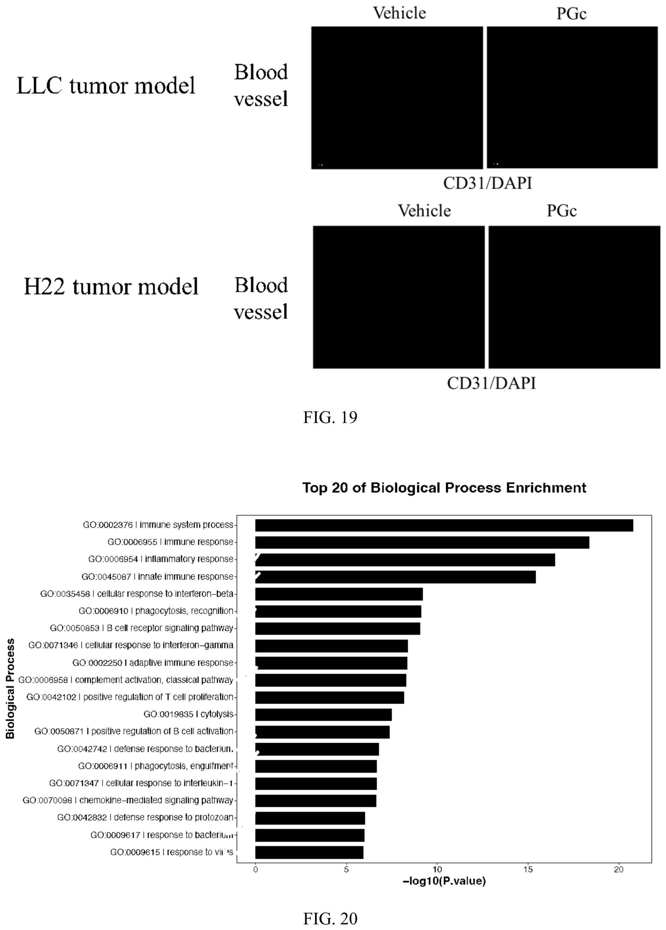

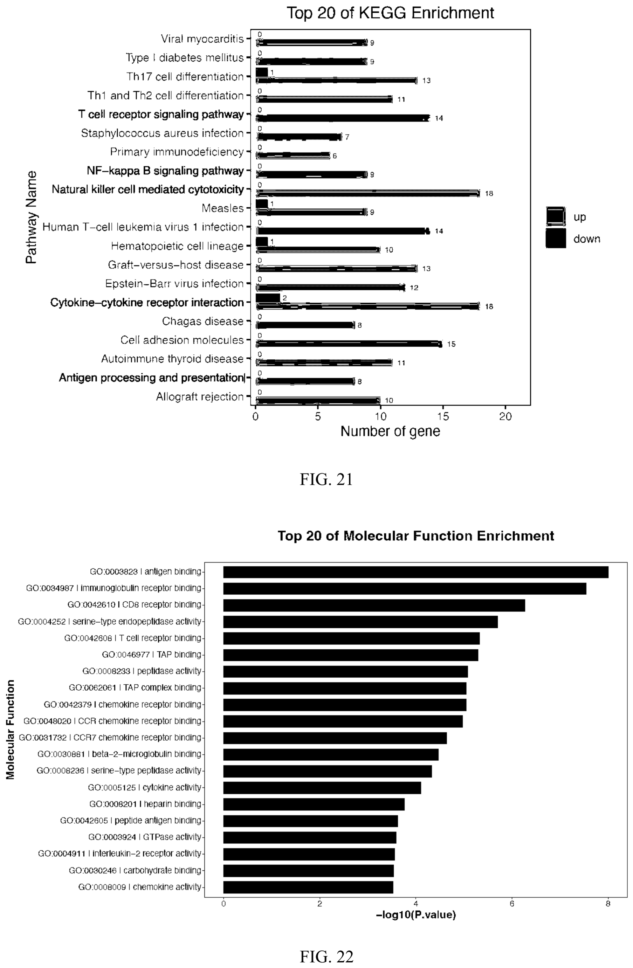

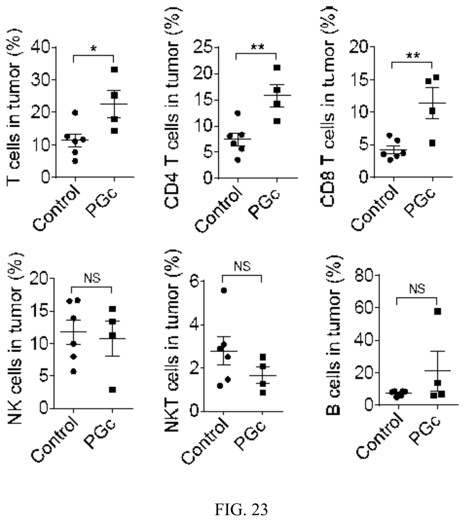

The formulation of the PGc preparation used in the animal models was identical to that described in Example 1.
The procedure for establishing the LLC tumor model (LLC tumor-bearing mouse model) was the same as that described in Example 1.
The method for constructing the H22 tumor model (H22 tumor-bearing mouse model) was the same as that described in Example 2.
Cell staining was performed using standard techniques well known in the field and will not be elaborated here.
RNA-seq experiments were outsourced to a third-party service provider.
The results were shown in FIGs. 16-23. Pathological analysis and RNA sequencing data indicated the following mechanistic effects of PGc: (1) Promotion of M1 macrophage activation (FIGs. 16-17); (2) Enhancement of T-cell activation (FIGs. 18 and 23); (3) Inhibition of angiogenesis within tumor tissues (FIG. 19); (4) Comprehensive activation and effective coordination of both innate and adaptive immune systems (FIGs. 20-22).
Note: The previous conclusion of no significant difference in CD8 in Example 7 was based on results from an initial single experiment. Subsequent repeated experiments, as well as the experiment in this example, consistently demonstrated significant changes in CD8.
The humanized PBMC HCC827 lung cancer mouse model demonstrated that the comprehensive tumor inhibition rate of the PGc biological injection reached 68.515%. In the H22 hepatocellular carcinoma model established in C57BL/6 mice, the tumor inhibition rate, when the PGc biological injection was used alone, was 89.8%. In the melanoma metastasis model, the PGc biological injection exhibited a remarkable inhibitory effect on melanoma metastasis. In the atherosclerosis mouse model, PGc showed a significant alleviation in atherosclerosis. In the HPV mouse model, PGc demonstrated notable therapeutic efficacy, effectively converting HPV-positive status to HPV-negative.
The above examples are provided to illustrate the disclosed embodiments of the present disclosure and shall not be construed as limiting the present disclosure. Furthermore, various modifications listed herein and changes to the methods in the present disclosure will be apparent to those skilled in the art without departing from the scope and spirit of the present disclosure. Although the present disclosure has been specifically described in conjunction with various specific preferred embodiments, it should be understood that the present disclosure shall not be limited to these specific embodiments. In fact, various modifications that are apparent to those skilled in the art as described above for implementing the present disclosure shall all be included within the scope of the present disclosure.

**Claims**

1. A pharmaceutical composition, comprising a first active ingredient, a second active ingredient, and a pharmaceutically acceptable carrier or excipient; wherein the first active ingredient is a microbial agent comprising one or more of *Staphylococcus aureus, Bordetella pertussis,* diphtheria toxoid, tetanus toxoid, *Salmonella typhi,* and *Salmonella paratyphi;* wherein the second active ingredient comprises polyinosinic acid, polycytidylic acid, and vitamin A.

2. The pharmaceutical composition according to claim 1, wherein the microbial agent is an inactivated preparation, and/or the microbial agent is a liquid preparation or a solid preparation.

3. The pharmaceutical composition according to claim 1, wherein the pharmaceutical composition further comprises one or more of the following:

   1) based on a total volume of the pharmaceutical composition, a concentration of *Staphylococcus aureus* is in a range of $2\times10^7$-$3\times10^9$ CFU/mL;
   2) based on the total volume of the pharmaceutical composition, a concentration of *Bordetella pertussis* is in a range of $7\times10^7$-$9\times10^9$ CFU/mL;
   3) based on the total volume of the pharmaceutical composition, a concentration of the diphtheria toxoid is in a range of 1 LF-5 LF/mL;
   4) based on the total volume of the pharmaceutical composition, a concentration of the tetanus toxoid is in a range of 0.1 LF-5 LF/mL;
   5) based on the total volume of the pharmaceutical composition, a concentration of *Salmonella typhi* is in a range of $1.5\times10^6$-$5\times10^8$ CFU/mL;
   6) based on the total volume of the pharmaceutical composition, a concentration of *Salmonella paratyphi* is in a range of $1\times10^6$-$3\times10^8$ CFU/mL.

4. The pharmaceutical composition according to claim 1, wherein the *Salmonella paratyphi* is one or more of *Salmonella paratyphi A, Salmonella paratyphi B,* and *Salmonella paratyphi C*; preferably, the *Salmonella paratyphi* is one or more of *Salmonella paratyphi A* and *Salmonella paratyphi B;* more preferably, based on a total volume of the pharmaceutical composition, a concentration of *Salmonella paratyphi A* is in a range of $0.1 \times 10^7$-$8 \times 10^7$ CFU/mL, and/or a concentration of *Salmonella paratyphi B* is in a range of $0.1 \times 10^7$-$8 \times 10^7$ CFU/mL.

5. The pharmaceutical composition according to claim 1, wherein the polyinosinic acid and the polycytidylic acid are, respectively, selected from a polymer formed solely from inosinic acid, a polymer formed solely from cytidylic acid, and a copolymer of inosinic acid and cytidylic acid; preferably, when the polyinosinic acid and the polycytidylic acid are a polymer formed solely from inosinic acid and a polymer formed solely from cytidylic acid, respectively, a mass ratio of the polyinosinic acid to the polycytidylic acid is in a range of 1:0.1-1:10; more preferably, the mass ratio of the polyinosinic acid to the polycytidylic acid is 1:1; more preferably, based on a total volume of the pharmaceutical composition, concentrations of the polyinosinic acid and the polycytidylic acid are each less than 0.5g/100mL; even more preferably, the concentrations of the polyinosinic acid and the polycytidylic acid are each in a range of 0.01g/100mL-0.5g/100mL.

6. The pharmaceutical composition according to claim 1, wherein the vitamin A is vitamin A1 and/or vitamin A2; more preferably, based on a total volume of the pharmaceutical composition, a concentration of the vitamin A is less than 1g/100mL.

7. The pharmaceutical composition according to claim 1, wherein the pharmaceutically acceptable carrier or excipient comprises sodium carboxymethyl cellulose, aluminum stearate, Tween 80, lecithin, soybean oil, dextran, fat emulsion, and water; preferably, the aluminum stearate is aluminum monostearate or aluminum distearate.

8. The pharmaceutical composition according to claim 7, wherein the pharmaceutical composition further comprises one or more of the following:

1) based on a total volume of the pharmaceutical composition, a concentration of the sodium carboxymethyl cellulose in the pharmaceutically acceptable carrier or excipient is less than 2g/100mL; preferably, based on the total volume of the pharmaceutical composition, the concentration of the sodium carboxymethyl cellulose in the pharmaceutically acceptable carrier or excipient is in a range of 0.2-2g/100mL;
2) based on the total volume of the pharmaceutical composition, a concentration of the aluminum stearate in the pharmaceutically acceptable carrier or excipient is less than 3g/100mL; preferably, based on the total volume of the pharmaceutical composition, the concentration of the aluminum stearate in the pharmaceutically acceptable carrier or excipient is in a range of 0.3-3g/100mL;
3) based on the total volume of the pharmaceutical composition, a concentration of the Tween 80 in the pharmaceutically acceptable carrier or excipient is less than 1mL/100mL; preferably, based on the total volume of the pharmaceutical composition, the concentration of the Tween 80 in the pharmaceutically acceptable carrier or excipient is in a range of 0.1-1mL/100mL;
4) based on the total volume of the pharmaceutical composition, a concentration of the lecithin in the pharmaceutically acceptable carrier or excipient is more than 50mg/100mL; preferably, based on the total volume of the pharmaceutical composition, the concentration of the lecithin in the pharmaceutically acceptable carrier or excipient is in a range of 50-2000mg/100mL;
5) based on the total volume of the pharmaceutical composition, a concentration of the soybean oil in the pharmaceutically acceptable carrier or excipient is less than 15mL/100mL; preferably, based on the total volume of the pharmaceutical composition, the concentration of the soybean oil in the pharmaceutically acceptable carrier or excipient is in a range of 1-15mL/100mL;
6) based on the total volume of the pharmaceutical composition, a concentration of the dextran in the pharmaceutically acceptable carrier or excipient is less than 10g/100mL; preferably, based on the total volume of the pharmaceutical composition, the concentration of the dextran in the pharmaceutically acceptable carrier or excipient is in a range of 1-10g/100mL;
7) based on the total volume of the pharmaceutical composition, a concentration of the fat emulsion in the pharmaceutically acceptable carrier or excipient is more than 10mL/100mL; preferably, based on the total volume of the pharmaceutical composition, the concentration of the fat emulsion in the pharmaceutically acceptable carrier or excipient is in a range of 10-80mL/100mL.

9. The pharmaceutical composition according to claim 1, wherein the pharmaceutical composition is an injection.

10. The pharmaceutical composition according to any one of claims 1-9 for use in the preparation of a product for treating, preventing, or diagnosing a disease.

11. The use according to claim 10, wherein the disease is selected from cancer, arteriosclerosis, HPV infection, and atrophic gastritis; preferably, the cancer is selected from liver cancer, lung cancer, melanoma, colorectal cancer, gastric cancer, ovarian cancer, cholangiocarcinoma, cervical cancer, pancreatic cancer, prostate cancer, sarcoma, and breast cancer.

Normal Saline
Cyclophosphamide
PGa
PGb
PGc
PGd

FIG. 1

FIG. 2

FIG. 3

(Front)    (Back)          (Front)    (Back)

NS group              PGc group

FIG. 4

EP 4 706 672 A1

Day14 Blood sample

NCG , Female

Dosing

-Day7

-Day1  Day0  Day4  Day8  Day12  Day16

PBMC,
5E6, i.p.

HCC827,
5E6, s.c.

FIG. 5

FIG. 6

FIG. 7

FIG. 8

FIG. 9

FIG. 10

FIG. 11

FIG. 12

FIG. 13

Tumor weight:

A 39 : 0.63    0.43    0.20    0.18    0.17    0.16

B 39 : 0.31    0.30    0.15    0.10    0.08    0.02

FIG. 14

FIG. 15

FIG. 16

EP 4 706 672 A1

FIG. 17

FIG. 18

FIG. 19

**Top 20 of Biological Process Enrichment**

FIG. 20

## Top 20 of KEGG Enrichment

FIG. 21

## Top 20 of Molecular Function Enrichment

FIG. 22

FIG. 23

## INTERNATIONAL SEARCH REPORT

| | International application No. |
|---|---|
| | **PCT/CN2024/089764** |

### A. CLASSIFICATION OF SUBJECT MATTER

A61K39/00(2006.01)i; A61K35/74(2015.01)i; A61K31/07(2006.01)i; A61K47/34(2017.01)i; A61P35/00(2006.01)i

According to International Patent Classification (IPC) or to both national classification and IPC

### B. FIELDS SEARCHED

Minimum documentation searched (classification system followed by classification symbols)

IPC: A61K; A61P

Documentation searched other than minimum documentation to the extent that such documents are included in the fields searched

Electronic data base consulted during the international search (name of data base and, where practicable, search terms used)

CNABS, CNTXT, VEN, DWPI, USTXT, WOTXT, EPTXT, CNKI: 普公生物科技, 常健, 陈磊, 高文斌, 金黄色葡萄球菌, 百日咳杆菌, 白喉类毒素, 破伤风类毒素, 伤寒杆菌, 副伤寒甲杆菌, 副伤寒乙杆菌, 聚肌苷酸, 聚胞苷酸, 维生素A, 灭活, 卵磷脂, staphylococcus aureus, bordetella pertussis, diphtheria toxoid, tetanus toxin, salmonella typhi, paratyphoid, polyinosinic, polycytidylic, poly(i: c), vitamin A, retin+, lecithin

### C. DOCUMENTS CONSIDERED TO BE RELEVANT

| Category* | Citation of document, with indication, where appropriate, of the relevant passages | Relevant to claim No. |
|---|---|---|
| Y | CN 106667907 A (SUN REN'E) 17 May 2017 (2017-05-17) description, paragraphs [0015]-[0021], and embodiment 9 | 1-11 |
| Y | ZHUANG, Huijing et al. "Immunomodulator polyinosinic-polycytidylic acid enhances the inhibitory effect of 13-cis-retinoic acid on neuroblastoma through a TLR3-related immunogenic-apoptotic response" *Laboratory Investigation*, Vol. 100, No. 4, 19 December 2019 (2019-12-19), pages 606-618 | 1-11 |
| Y | SZABO, A. et al. "Temporally designed treatment of melanoma cells by ATRA and polyI: C results in enhanced chemokine and IFNβ secretion controlled differently by TLR3 and MDA5" *Melanoma Research*, Vol. 22, No. 5, 31 December 2012 (2012-12-31), pages 351-361 | 1-11 |
| A | CN 101669973 A (MA YIBING) 17 March 2010 (2010-03-17) claims 1-10 | 1-11 |

☑ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **26 July 2024** | **09 August 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

# EP 4 706 672 A1

## C.    DOCUMENTS CONSIDERED TO BE RELEVANT

| Category* | Citation of document, with indication, where appropriate, of the relevant passages | Relevant to claim No. |
|---|---|---|
| A | CN 112618581 A (PUGONG BIOTECHNOLOGY (HANGZHOU) CO., LTD.) 09 April 2021 (2021-04-09)<br>  claims 1-12 | 1-11 |
| A | CN 115702927 A (GUANGZHOU MEDICAL UNIVERSITY) 17 February 2023 (2023-02-17)<br>  claims 1-8 | 1-11 |
| A | CN 101569746 A (KONG RUNLIAN et al.) 04 November 2009 (2009-11-04)<br>  claims 1-10 | 1-11 |

Form PCT/ISA/210 (second sheet) (July 2022)

## INTERNATIONAL SEARCH REPORT
### Information on patent family members

| International application No. |
| --- |
| **PCT/CN2024/089764** |

| Patent document cited in search report | | | Publication date (day/month/year) | Patent family member(s) | Publication date (day/month/year) |
| --- | --- | --- | --- | --- | --- |
| CN | 106667907 | A | 17 May 2017 | None | |
| CN | 101669973 | A | 17 March 2010 | None | |
| CN | 112618581 | A | 09 April 2021 | None | |
| CN | 115702927 | A | 17 February 2023 | None | |
| CN | 101569746 | A | 04 November 2009 | None | |

Form PCT/ISA/210 (patent family annex) (July 2022)